# EUROPEAN PATENT SPECIFICATION

(11) **EP 2 931 883 B1**
(45) Date of publication and mention of the grant of the patent: **01.02.2017**
(21) Application number: 12809243.4
(22) Date of filing: 17.12.2012
(51) Int. Cl.: C12N 9/12, C07D 401/12, C07D 471/00

(54) **P38 MAPK INHIBITORS FOR THE TREATMENT OF INFLAMMATORY DISEASES**
P38 MAPK-INHIBITOREN ZUR BEHANDLUNG VON ENTZÜNDUNGSKRANKHEITEN
INHIBITEURS P38 MAPK POUR LE TRAITEMENT DES MALADIES INFLAMMATOIRES

(43) Date of publication of application: 21.10.2015
(73) Proprietor: Allinky Biopharma, 28049 Madrid (ES)
(72) Inventor: GÓMEZ, Patricia, E-28049 Madrid (ES); CARRASCO, Esther, E-28049 Madrid (ES); CAMPOS, Pedro, E-28049 Madrid (ES); DELEYTO, Patricia, E-28049 Madrid (ES); VEGA, Miguel, E-28049 Madrid (ES); GÓMEZ-REINO, Juan Jesús, E-28049 Madrid (ES); CONDE, Carmen, E-28049 Madrid (ES); GUALILLO, Oreste, E-28049 Madrid (ES); PÉREZ, Juan Jesús, E-28049 Madrid (ES); MESSEGUER, Ángel, E-28049 Madrid (ES)
(74) Representative: Hoffmann Eitle
(86) International application number: PCT/EP2012/075756
(87) International publication number: WO 2014/094816

(56) References cited:
- US-A1- 2003 078 432
- BOEHM J C ET AL: "New inhibitors of p38 kinase", EXPERT OPINION ON THERAPEUTIC PATENTS, INFORMA HEALTHCARE, GB, vol. 10, no. 1, 1 January 2000 (2000-01-01), pages 25-37, XP002259248, ISSN: 1354-3776, DOI: 10.1517/13543776.10.1.25
- TRIFILIEFF ALEXANDRE ET AL: "CGH2466, a combined adenosine receptor antagonist, p38 mitogen-activated protein kinase and phosphodiesterase type 4 inhibitor with potent in vitro and in vivo anti-inflammatory activities", BRITISH JOURNAL OF PHARMACOLOGY, vol. 144, no. 7, April 2005 (2005-04), pages 1002-1010, XP002673576, ISSN: 0007-1188
- PEIFER CHRISTIAN ET AL: "New approaches to the treatment of inflammatory disorders small molecule inhibitors of p38 MAP kinase", CURRENT TOPICS IN MEDICINAL CHEMISTRY, vol. 6, no. 2, 2006, pages 113-149, XP002673577, ISSN: 1568-0266
- AKELLA ET AL: "Unique MAP Kinase binding sites", BIOCHIMICA ET BIOPHYSICA ACTA (BBA) - PROTEINS & PROTEOMICS, ELSEVIER, NETHERLANDS, vol. 1784, no. 1, 19 November 2007 (2007-11-19), pages 48-55, XP022425221, ISSN: 1570-9639, DOI: 10.1016/J.BBAPAP.2007.09.016

## Description

### Fiend of the invention

The present invention provides new p38 mitogen activated protein (MAP) kinase allosteric inhibitors which are useful for the treatment of p38 mediated diseases such as inflammatory diseases, e.g. rheumatoid arthritis, osteoarthritis, psoriatic arthritis and pain.

### Background art

p38α MAP kinase (MAPK) is an intracellular serine/threonine kinase involved in the regulation of inflammatory cell signals and plays a central role in the regulation of pro-inflammatory cytokine production. Activation of p38α is produced by upstream kinases MKK6 and MKK3. At molecular level, like other protein kinases, p38α is responsible for the transfer of the γ-phosphate form ATP to a range of substrate proteins including the transcription factors ATF2, Elk-1 and MEF2A and downstream kinases like MK2, MK3, PRAK, MNK1/2 and MSK1, modulating their function (Stokoe et al., 1992, EMBO J. 11, 3985-3994).

p38α has been identified as a potential target for antiinflammatory drugs, and different binding sites for these drugs have been identified (Akella et al., January 2008, Biochim Biophys Acta; 1784(1); 48-55). Yong et al. review different p38 MAPK inhibitors which are under development as potential drugs for the treatment of inflammatory diseases and cancer (Yong et al., 2009, Expert Opin. Investig. Drugs; 18(12)). The majority of the drug candidates have proved to be competitive with ATP, binding to the active site. A few inhibitors have also been found that bind to a site adjacent to the active site. Akella et al. discuss the potential relevance of other binding sites such as the binding sites for D-motifs, FXFP and the Backside site.

For example, WO2008/140066 A2 describes pyridone derivative compounds as inhibitors of p38α MAPK, possessing analgesic, antiinflammatory and arthritis mutilans action.

EP 1 529 531 A1 describes oxadiazolopyrazine derivatives for the treatment protein kinase-dependent diseases such as inflammatory diseases.

### Summary of the Invention

It is the object underlying the present invention to provide compounds, compositions and formulations that are useful for the treatment of p38 mediated diseases (excluding tumour growth and metastasis (cancer)), such as inflammatory diseases, particularly chronic inflammatory diseases and sustained oxidative stress conditions, such as rheumatoid arthritis, osteoarthritis, dermatitis, fibrosis, neuritis, psoriasis and psoriatic arthritis as well as pain. Particularly, pain caused by an inflammatory process.

The present invention thus provides compounds for use in a method for treatment of inflammatory diseases, as well as for use in therapy in general, wherein the compound binds to the region composed of amino acids at positions 170 - 199 of Mitogen-activated protein kinase 14 (Uniprot accession nr Q16539 or SEQ ID No 1) and/or Mitogen-activated protein kinase 11 (Uniprot accession nr Q15759 or SEQ ID No 2), SEQ ID NO.1 and SEQ ID NO.2 being the amino acid sequences of MAPK14(p38α) and MAPK11(p38β), respectively. The specific region composed of amino acids at positions 170 - 199 is herein disclosed as SEQ ID NO.4 for Mitogen-activated protein kinase 14 and SEQ ID NO.5 for Mitogen-activated protein kinase 11 and are believed to be new inhibitory binding sites. Its three-dimensional structure is available from the Protein Data Bank (PDB entry 2OZA).

A first group of suitable compounds which can be used according to the present invention includes peptides comprising SEQ ID NO. 3 (Uniprot accession nr P49137) and having a length of up to 20 amino acids.

Suitable compounds also include small non-peptidyl molecules that mimic the 3D structure and charge distribution of the peptides of SEQ ID NO. 3.

In particular, the present invention provides compounds of the following formula: 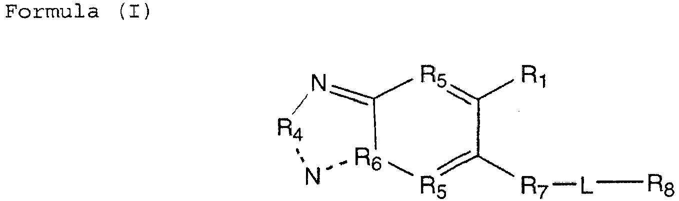 wherein
R1 is NR2R3, or OR2;
R2 is C5-C7-aryl or C5-C7-heteroaryl;
   wherein the C5-C7-aryl or C5-C7-heteroaryl, is substituted with one or more of a -OR10, -SR10, -NHCOR10, -CONHR10, -COR10, -COOR10 , -OCOR10, -NR10R11, -SO₂R10, -SO₂NR10R11, -CF3, -OCF3 or -CN, wherein R10 and R11 are independently of each other hydrogen, C1-C4-alkyl, C1-C4-alkenyl or C1-C4-alkynyl; and
   wherein if R1 is NR2R3, the C5-C7-aryl or C5-C7-heteroaryl, can be connected to the N via a single bond or NH;
R3 is hydrogen or a C1-C4-alkyl group;
R4---N---R6 is N=N-N or O-N=C;
R5 is independently of each other N or CR11; wherein R11 is hydrogen, OH, or SH;
R7 is an N-containing saturated or unsaturated C3-C7 heterocycloalkyl;
L is a linker L, preferably a single covalent bond (so that R7 is linked directly to R8) or a C1 to C3 alkylen group, wherein one of the CH2 groups may be replaced by O, NH, or S;
R8 is C5-C10-aryl or C5-C10-heteroaryl, which may be substituted at 4 position with a halogen, C1-C4 alky group, -OR12 or -SR12; wherein R12 is a C1 to C4 alkyl group;
or a pharmaceutically acceptable salt, ester, and amide thereof.

The present invention also provides methods of treatment using the p38 MAPK inhibitors described herein for the treatment of inflammatory diseases; a method for identifying an inhibitor; and a method of providing an inhibitor.

### Figures

**Fig. 1****:** Inhibitory action of the peptides of SEQ ID NO 3 and SEQ ID NO 6 depending on their concentration.
**Fig. 2** Inflammatory mediators (IL6,IL8 and MCP-1) in FLS stimulated with TNF-alpha in the presence of compounds 21011_07 and SB (positive control).
**Fig. 3****:** Nitric Oxide production (measuring Nitrate) in chondrocytes stimulated with LPS in the presence of compound 1.

### Detailed description

The present invention thus provides compounds for use
in a method for treatment of p38 mediated diseases such as inflammatory diseases, wherein the compound binds to the region composed of amino acids at positions 170 - 199 of SEQ ID NO.1 and/or SEQ ID NO.2, SEQ ID NO.1 and SEQ ID NO.2 being the amino acid sequences of MAPK14(p38α) and MAPK11(p38β), respectively. The compounds of the present invention have preferably an inhibitory effect on the protein of SEQ ID NO.1 and/or SEQ ID NO.2, but not on the mutant R186A or R189A (please refer to Example 2 for the generation of these two mutants) of the protein of SEQ ID NO.1 and/or SEQ ID NO.2.

Suitable compounds can be identified as follows.

The compounds to be used by the present invention are identified by *in silico* screening and a subsequent binding assay. Starting from a suitable database, such as ZINC (http://zinc.docking.org/), which is a free database of commercially available compounds, several selection criteria should be applied to identify candidates that are then subjected to the subsequent binding assay.

Firstly, the compound must fulfil a pharmacophore structure that is essentially similar to the one fulfilled by the peptides of SEQ ID NO.3. This peptide was found to fulfil the following pharmacophore, wherein five points are defined as follows:
Point A: a hydrophobic/aromatic moiety;
Point B: a hydrophobic/aromatic moiety;
Point C: a hydrogen acceptor point;
Point D: a hydrogen acceptor point; and
Optional: Point E: a hydrogen donor, wherein the spatial relation between the pharmacophore points are specified by spheres of different radii (r) centered at their Cartesian coordinates: point A (7.99, 34.86 40.16), r = 1.5; Point B (12.16; 36.69; 37.15), r= 1.6 ; Point C (7.03, 33.05, 40.27), r= 1.2; Point D (9.30, 37.74, 46.62), r=1.5; Point E (12.72, 36.93, 35.072), r= 1.6.

These criteria can be used as search criteria in the compound database such as ZINC.

The process for assessing whether a new compound fulfils this pharmacophore requirement is done in a hierarchical manner. In order to make the process more efficient, the pharmacophore is transformed in a set of distance constraints between pairs of chemical moieties: 2.36<d(A,B)<8.56; 4.22<d(B.C)<9.82; 5.5.1<d(C,D)<10.91; 8.97<d(D,E)<15.17; -0.65<d(A,C)<4.75; 6.84<d(B,D)<13.04; 5.83<d(C,E)<11.43; 4.09<d(A,D)<10.29; -1.03<d(B,E)<5.37; 4.15d(A,E)<10.35. In other words, the spatial relation (in A) between the pharmacophore points is defined as follows: 2.36<d(A,B)<8.56; 4.22<d(B,C)<9.82; 5.51<d(C,D)<10.91; 8.97<d(D,E)<15.17; 0.65<d(A,C)<4.75; 6.84<d(B,D)<13.04; 5.83<d(C,E)<11.43; 4.09<d(A,D)<10.29; 1.03<d(B,E)<5.37; 4.15<d((A,E)<10.35.

First, the different chemical moieties are identified. For a compound to be considered for a further study, it should exhibit at least four of the requirements of the pharmacophore. Then, the distances between moieties in the molecule are compared with those of the pharmacophore. Finally, if the distances fulfill pharmacophore requirements, molecules are superimposed with the pharmacophore through geometrical superimposition. The root-mean-square-deviation is then used to assess the degree of fulfillment of the pharmacophore.

A second selection rule is that the molecular mass of the compound should be not greater than 500 Dalton. Preferably, the compound complies with the so-called Lipinski's rules, which states that an orally active drug has no more than one violation of the following criteria:
- Not more than 5 hydrogen bond donors (nitrogen or oxygen atoms with one or more hydrogen atoms)
- Not more than 10 hydrogen acceptors (nitrogen or oxygen atoms)
- A molecular mass not greater than 500 Dalton
- An octanol-water partition coefficient log P not greater than 5

The hits obtained by *in silico* screening are then subjected to a p38 binding assay. If the compound shows an inhibitory action on the protein of SEQ ID NO.1 and/or SEQ ID NO.2, but to a lesser extent on the mutant R186A or R189A of the protein of SEQ ID NO.1 and/or SEQ ID NO.2, and if this is achieved in an essentially ATP concentration independent manner, the claimed compound is one that binds to the region composed of amino acids at positions 170 - 199 of SEQ ID NO.1 and/or SEQ ID NO.2 and can thus be used for therapy according to the present invention.

The compounds have preferably an IC50 of 0.01 nM to 100 µM for inhibiting the MAPK p38 protein activity.

Compounds that bind to the region composed of amino acids at positions 170 - 199 of SEQ ID NO.1 and/or SEQ ID NO.2 can be structurally rather diverse.

A first group of compounds are peptides comprising SEQ ID NO. 3 and having a length of up to 20 amino acids, preferably up to 10 amino acids. Most preferred are peptides consisting of the amino acid sequence of SEQ ID NO 3 (Tyr Ser Asn His Gly Leu) and or of SEQ ID NO 6 (Phe Tyr Ser Asn His Gly Leu). These peptides fulfil the above stated pharmacophore criteria because the imidazole group of the His and the aliphatic side chain of the Asn act as hydrophobic/aromatic groups (A and B points of the pharmacophore). Moreover, the carbonyl group in the backbone of the Leu fulfils points C and D of the pharmacophore, respectively. Finally the NH moiety of His corresponds to point E of the pharmacophore.

These peptides can be prepared using standard solid or liquid phase peptide synthesis protocols.

A second group of compounds are compounds of the following formula: 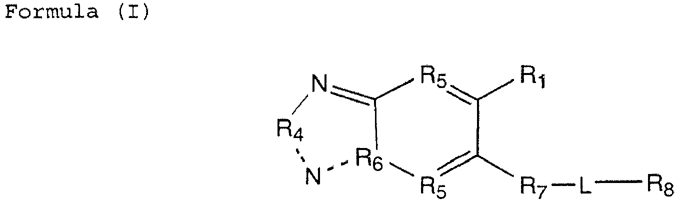 wherein
R1 is NR2R3, or OR2;
**R2** is C5-C7-aryl or C5-C7-heteroaryl;
   wherein the C5-C7-aryl or C5-C7-heteroaryl, group is substituted with one or more of a -OR10, -SR10, -NHCOR10, -CONHR10, -COR10, -COOR10 , -OCOR10, -NR10R11, -SO2R10 -SO2NR10R11, -CF3, -OCF3 or -CN, wherein R10 and R11 are independently of each other hydrogen, C1-C4-alkyl, C1-C4-alkenyl or C1-C4-alkynyl; and
   wherein if R1 is NR2R3, the C5-C7-aryl or C5-C7-heteroaryl, can be connected to the N via a single bond or NH;
**R3** is hydrogen or a C1-C4-alkyl group;
**R4---N---R6** is N=N-N or O-N=C;
**R5** is independently of each other N or CR11; wherein R11 is hydrogen, OH, or SH;
**R7** is an N-containing saturated or unsaturated C3-C7 heterocycloalkyl;
**L** is a linker L, preferably a single covalent bond (so that R7 is linked directly to R8) or a C1 to C3 alkylen group, wherein one of the CH2 groups may be replaced by O, NH, or S;
**R8** is C5-C10-aryl or C5-C10-heteroaryl, which may be substituted at 4 position with a halogen, C1-C4 alky group, -OR12 or -SR12; wherein R12 is a C1 to C4 alkyl group;
or a pharmaceutically acceptable salt, ester, and amide thereof.

**R1** is preferably NR2R3.

**R2** is preferably
C6-arylor C5-C6-heteroaryl, which is substituted with one of a -OR10, -SR10, -NHCOR10, -CONHR10, - COR10, -COOR10 , -OCOR10, -NR10R11, -SO₂R10, -SO₂NR10R11, -CF3, - OCF3 or -CN, wherein R10 and R11 are independently of each other hydrogen, C1-C4-alkyl, C1-C4-alkenyl or C1-C4-alkynyl; particularly preferred is a substitution with -OR10
or -COOR10 (particularly preferred for R2 being C5-C5-heterocycloalkyl, C6-aryl, C5-C6-heteroaryl; in case of a six-membered ring, the substitution is preferably at position 4, and in case of a five-membered ring, the substitution is preferably at position 2), wherein R10 is hydrogen or C1-C4-alkyl; and wherein if R1 is NR2R3,
C5-C7-aryl, or C5-C7-heteroaryl, can be connected to the N via a single bond or NH;
**R3** is preferably H.
**R4---N---R6** is N=N-N or O-N=C.
**R5** is independently of each other N or CR11, wherein R11 is hydrogen;
**R7** is preferably an N-containing saturated C6 heterocycloalkyl.
L is preferably a C1 to C3 alkylen group, wherein one of the CH2 groups may be replaced by O, NH, S or CO;
**R8** is preferably C6-C10-aryl, or a C6-C10-heteroaryl group.

With regard to these preferred meanings of R1, R2, R3, R4---N-R6, R5, R7, R8 and L, it is particularly preferred to combine 2 of these preferred meanings, even more preferred being the combination of 3, 4, 5, 6, 7, or 8 of these preferred meanings.

Compounds of Formula I fulfil the pharmacophore requirements as illustrated below:

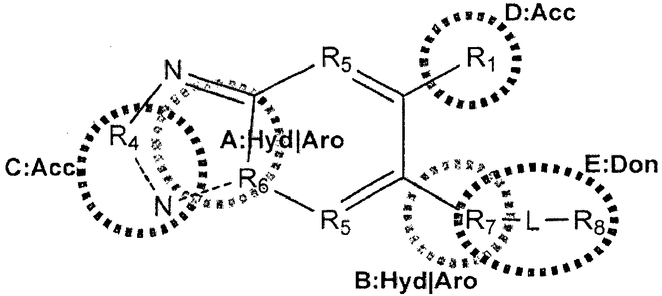

In one preferred -R7-L-R8 is 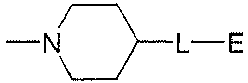 with L being the linker and E being the phenyl ring or the heterocycle.

In another preferred embodiment, -R7-L-R8 is 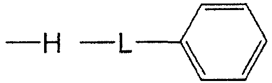 wherein H is an N-containing heterocycle with 5, 6, or 7 ring members, which may be saturated or non-saturated, and which may contain, besides N and CH2/CH, O, S, or N/NH as ring forming elements. Particularly preferred as -R7-L-R8 is

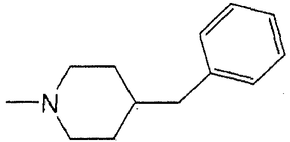

A preferred group of compounds of Formula I are those of Formula II: 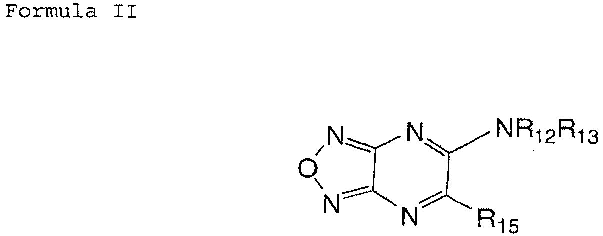 wherein
R12 is hydrogen, or a C1 to C4 alkyl group;
R13 is a ring structure,
the ring structure being an aryl ring which is substituted at positions or 4 with, CF3, OCF3, OR14, NHCOR14, COR14, CONHR14 or COOR14, wherein R14 is a C1 to C8 alkyl group;
or an O-, N- and/or S-containing heterocyclic, saturated or non-saturated ring with 5 to 7 ring members which is substituted at 3 or 4 position with a halogen atom, CF3, or OCF3, OR14, NHCOR14, COR14, or COOR14, wherein R14 is a C1 to C8 alkyl group, and which may be substituted with a halogen atom, CF3, OCF3, a C1 to C4 alkyl group, OR14, NHCOR14,COR14,or COOR14, wherein R14 is H or a C1 to C8 alkyl group at the remaining positions;
   wherein the ring structure or the O-, N- and/or S-containing heterocyclic can be connected to the N via a single bond or NH;
R15 is an N-containing heterocycle, with the N binding to the oxazolidinone ring structure, which is linked, via a linker, to either
a C6-C10-aryl or a phenyl ring which might be substituted at 4 position with OR14, NHCOR14, COR14, COOR14, wherein R14 is a C1 to C8 alkyl group, a halogen atom, CF3, or OCF3, and which may be substituted with a C1 to C4 alkyl group, a halogen atom, OR14, NHCOR14, COR14, COOR14, wherein R14 is a C1 to C8 alkyl group a halogen atom CF3, or OCF3 at the remaining positions;
or an O-, N- and/or S-containing heterocyclic, saturated or non-saturated ring with 5 to 7 ring members, and which may be substituted with a C1 to C4 alkyl group, OR14, NHCOR14, COR14, COOR14, wherein R14 is a C1 to C8 alkyl group a halogen atom CF3, or OCF3 at the remaining positions.

The most preferred substituent R13 is

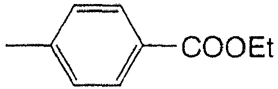

Preferred compounds include the following compounds: 1, 13-21, 23, 25,28-31 and 33.

For reference purposes, structures of the compounds 12, 22, 24, 26, 27 and 32 (reference compounds) are also shown.

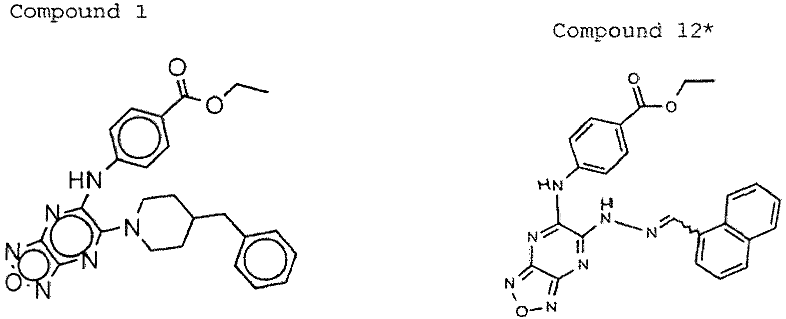

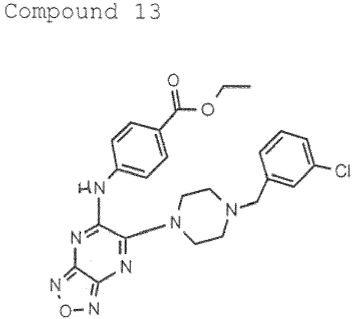

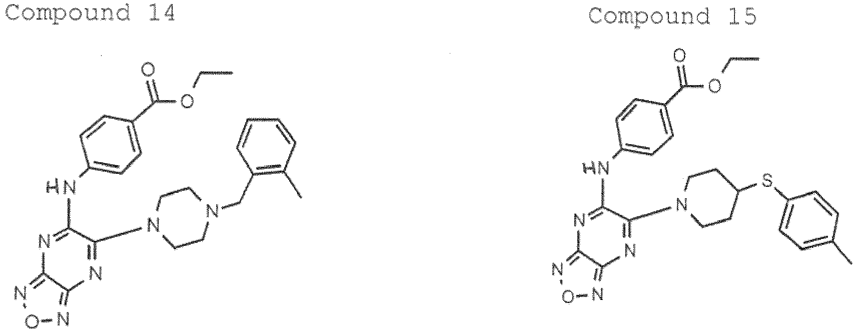

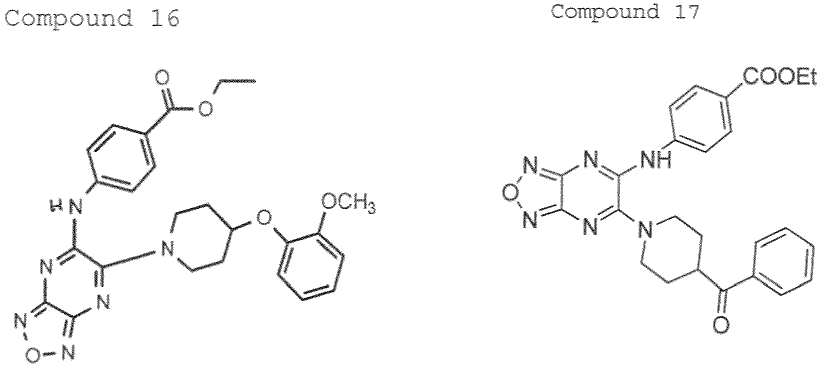

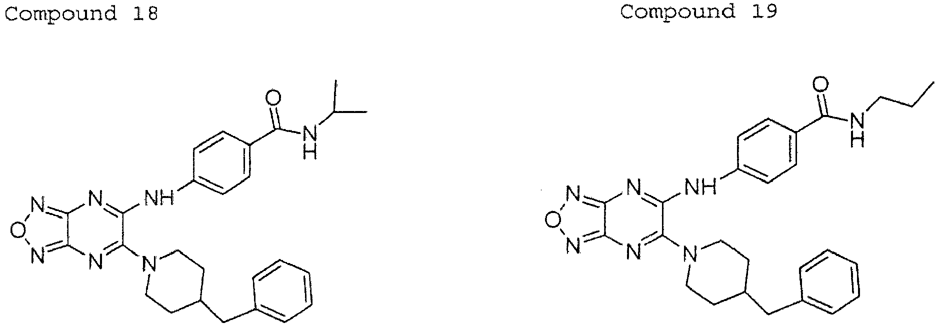

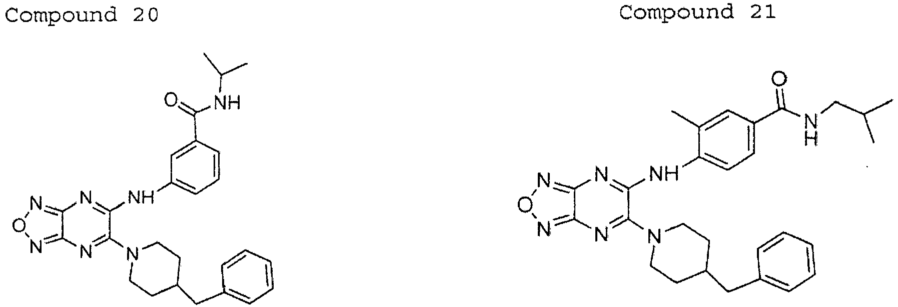

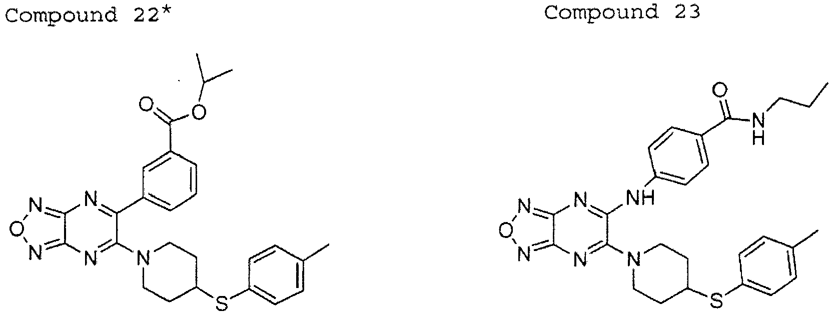

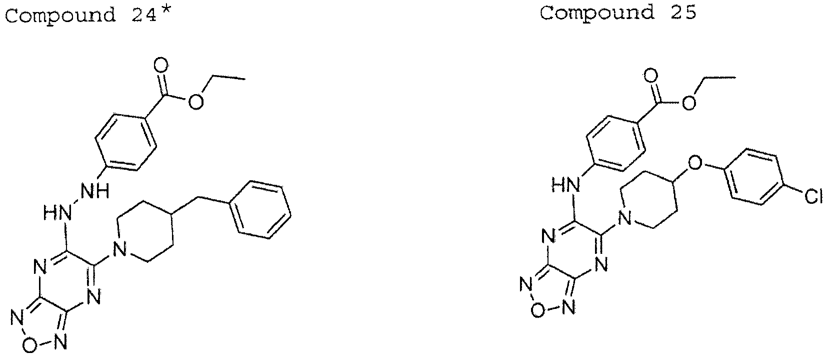

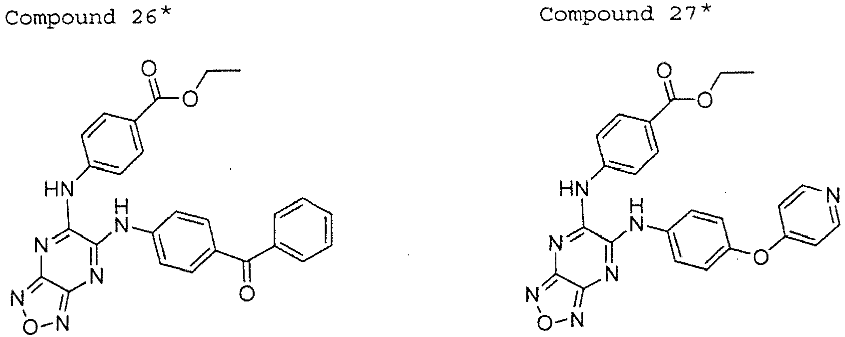

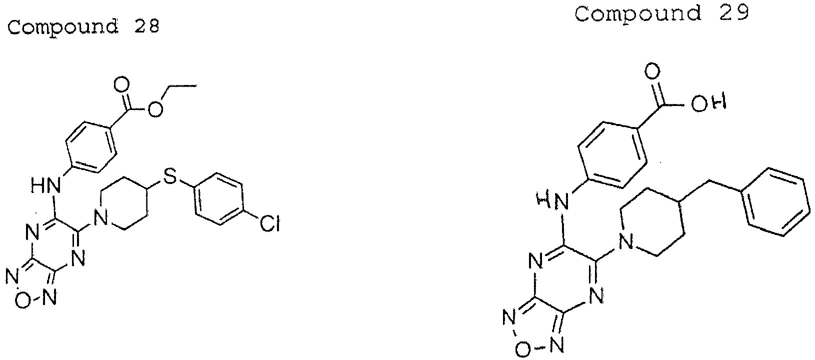

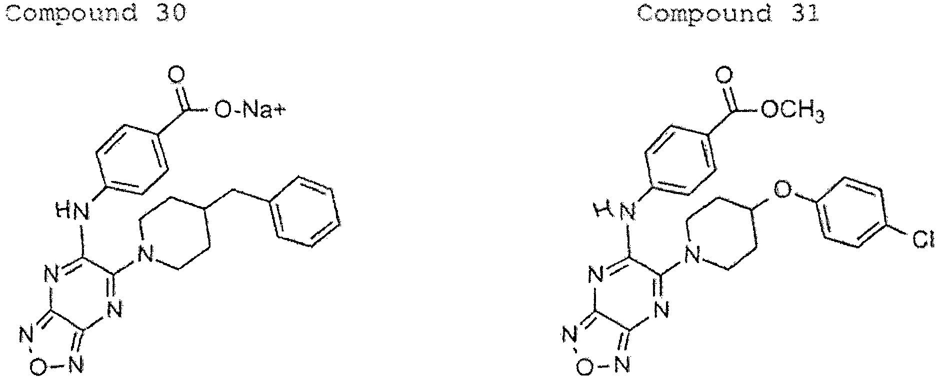

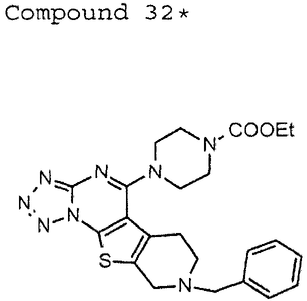

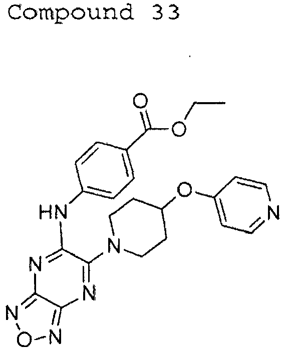

### *Reference Compounds

The present invention also provides the use of the compounds of formula I for therapy, such as for the treatment of p38 mediated diseases and disorders, such as inflammatory diseases and disorders.

The compounds of Formula I can be prepared essentially as described by Fernandez et al., 2002, Tetrahedron Letters 43, 4741-4745; Starchenkov et al., Chemistry of Heterocyclic Compounds 1997, 33(19), 1219-1233; and Khim.Geterotskil.Soedin.1997, 1402-1416. Compound 1 is for example available from Akos GmbH (reference AKOS001630569; http://www.akosgmbh.eu).

Other inhibitors (reference compounds), show the following structures

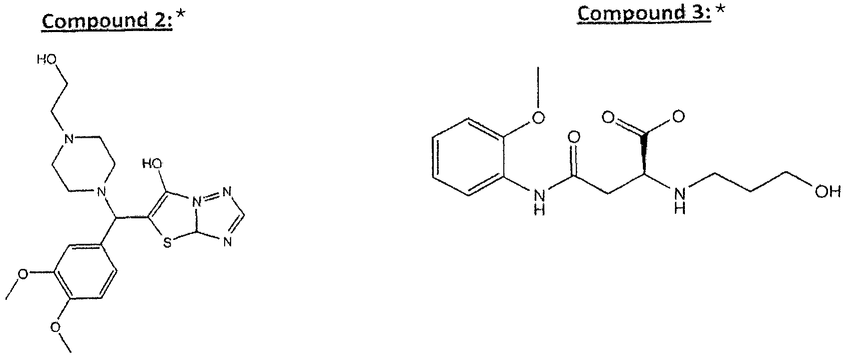

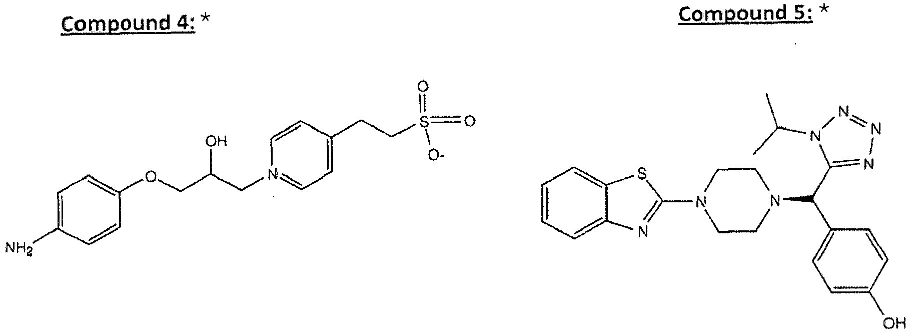

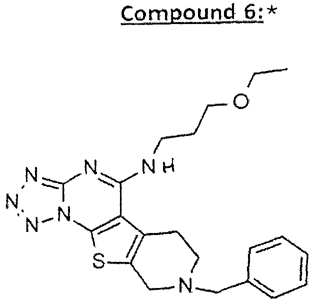

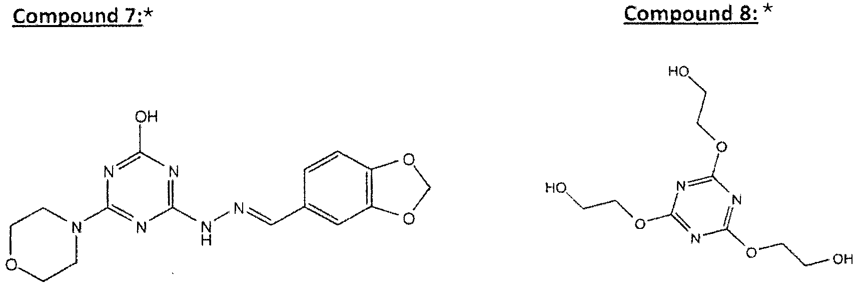

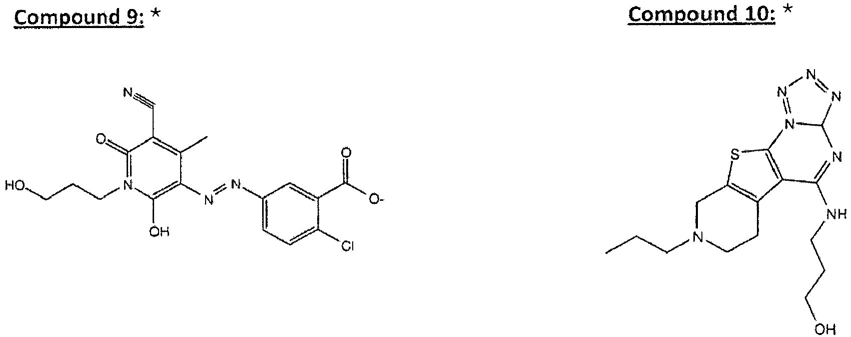

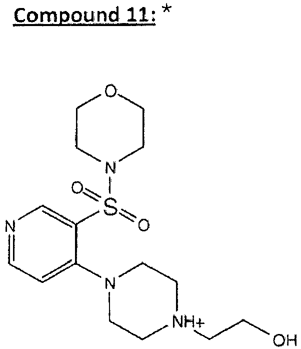

### *Reference Compounds

The compounds of the invention include pharmaceutically acceptable salts, esters, and amides therof, including but not limited to carboxylate salts, amino acid addition salts, esters,and amides of the compounds of the present invention which are, within the scope of sound medical judgment, suitable for use in contact with the tissues of patients without undue toxicity, irritation, allergic response, and the like, commensurate with a reasonable benefit/risk ratio, and effective for their intended use, as well as the zwitterionic forms, where possible, of the compounds of the invention. The term "salts" refers to the relatively non-toxic, inorganic and organic acid addition salts of compounds of the present invention. These salts can be prepared in situ during the final isolation and purification of the compounds or by separately reacting the purified compound in its free base form with a suitable organic or inorganic acid and isolating the salt thus formed. Representative salts include the hydrobromide, hydrochloride, sulfate, bisulfate, nitrate, acetate, oxalate, valerate, oleate, palmitate, stearate, laurate, borate, benzoate, lactate, phosphate, tosylate, citrate, maleate, fumarate, succinate, tartrate, naphthylate mesylate, glucoheptonate, lactobsonate, and laurylsulphonate salts, and the like. These may include cations based on the alkali and alkaline earth metals, such as sodium, lithium, potassium, calcium, magnesium, and the like, as well as non-toxic ammonium, quaternary ammonium, and amine cations including, but not limited to ammonium, tetramethylammonium, tetraethylammonium, methylamine, dimethylamine, trimethylamine, triethylamine, ethylamine, and the like, (See, for example, Berge S.M, et al, "Pharmaceutical Salts," J.Pharm.Sci., 1977;66:1-19 which is incorporated herein by reference.)

Accordingly, a further aspect of the present invention includes pharmaceutical compositions comprising as one or more compounds of the invention disclosed above, associated with a pharmaceutically acceptable carrier. For administration, the compounds are ordinarily combined with one or more adjuvants appropriate for the indicated route of administration. The compounds may be admixed with lactose, sucrose, starch powder, cellulose esters of alkanoic acids, stearic acid, talc, magnesium stearate, magnesium oxide, sodium and calcium salts of phosphoric and sulfuric acids, acacia, gelatin, sodium alginate, polyvinylpyrrolidine, and/or polyvinyl alcohol, and tableted or encapsulated for conventional administration. Alternatively, the compounds of this invention may be dissolved in saline, water, polyethylene glycol, propylene glycol, carboxymethyl cellulose colloidal solutions, ethanol, corn oil, peanut oil, cottonseed oil, sesame oil, tragacanth gum, and/or various buffers. Other adjuvants and modes of administration are well known in the pharmaceutical art. The carrier or diluent may include time delay material, such as glyceryl monostearate or glyceryl distearate alone or with a wax, or other materials well known in the art.

Examples of pharmaceutically acceptable, non-toxic esters of the compounds of this invention include C1-C6 alkyl esters, wherein the alkyl group is a straight or branched substituted or unsubstituted, C5-C7 cycloalkyl esters, as well as arylalkyl esters such as benzyl and triphenylmethyl. C1-C4 esters are preferred, such as methyl, ethyl, 2,2,2-trichloroethyl, and tert-butyl. Esters of the compounds of the present invention may be prepared according to conventional methods. Examples of pharmaceutically acceptable, non-toxic amides of the compounds of this invention include amides derived from ammonia, primary C1-C6 alkyl amines and secondary C1-C6 dialkyl amines, wherein the alkyl groups are straight or branched. In the case of secondary amines, the amine may also be in the form, of a 5- or 6-membered heterocycle containing one nitrogen atom. Amides derived from ammonia, C1-C3 alkyl primary amines and C,-C2 dialkyl secondary amines are preferred. Amides of the compounds of the invention may be prepared according to conventional methods.

These compounds can be administered individually or in combination, usually in the form of a pharmaceutical composition. Such compositions are prepared in a manner well known in the pharmaceutical art and comprise at least one active compound.

Accordingly, a further aspect of the present invention includes pharmaceutical compositions comprising as one or more compounds of the invention disclosed above, associated with a pharmaceutically acceptable carrier. For administration, the compounds are ordinarily combined with one or more adjuvants appropriate for the indicated route of administration. The compounds may be admixed with lactose, sucrose, starch powder, cellulose esters of alkanoic acids, stearic acid, talc, magnesium stearate, magnesium oxide, sodium alginate, polyvinylpyrrolidine, and/or polyvinyl alcohol, and tableted or encapsulated for conventional administration. Alternatively, the compounds of this invention may be dissolved in saline, water, polyethylene glycol, propylene glycol, carboxymethyl cellulose colloidal solutions, ethanol, corn oil, peanut oil, cottonseed oil, sesame oil, tragacanth gum, and/or various buffers. Other adjuvants and modes of administration are well known in the pharmaceutical art. The carrier or diluent may include time delay material, such as glyceryl monostearate or glyceryl distearate alone or with a wax, or other materials well known in the art.

The compounds and compositions of the present invention are useful for the treatment, prevention or amelioration of one or more symptoms of p38 kinase mediated diseases and disorders, including, but not limited to inflammatory diseases and disorders excluding tumor growth and metastasis (cancer). Particularly the compounds under Markush formula are useful for acute and chronic inflammatory diseases such as rheumatoid arthritis, osteoarthritis, dermatitis, fibrosis, psoriasis, psoriatic arthritis, musculoskeletal system inflammation and musculoskeletal system aging as well as pain, particularly inflammatory pain.

Other p38 mediated diseases for treatment with compounds include Reiter's syndrome, gout, traumatic arthritis, rubella arthritis, acute synovitis, rheumatoid spondylitis, gouty arthritis and other arthritic conditions, sepsis, septic shock, endotoxic shock, gram negative sepsis, toxic shoxk syndrome, Alzheimer's disease, stroke, ischemic and hemorrhagic stroke, neurotrauma head or spine injury, asthma, adult respiratory distress syndrome, chronic obstructive pulmonary disease, cerebral malaria, meningitis, chronic pulmonary inflammatory disease, silicosis, pulmonary sarcostosis, bone resorption disease, osteoporosis, restenosis, cardiac reperfusion injury, brain and renal reperfusion injury, chronic renal failure, thrombosis, glomerularonephritis, diabetes, non-insulin dependent diabetes, diabetic retinophaty, macular degeneration, graft vs host reaction, allograft rejection, inflammatory bowel disease, Chron's disease, ulcerative colitis, neurodegenerative disease, multiple sclerosis, Amyotrophic lateral sclerosis, diabetic retinopathy, macular degeneration, rhinovirus infection, gingivitis and periodontitis, eczema, contact dermatitis and conjunctivitis.

The present invention also provides a method for identifying compounds that can be used according to the present invention. Hence, the present invention provides a method for screening a compound that binds to the region composed of amino acids at positions 170 - 199 of SEQ ID NO.1 and/or SEQ ID NO.2, wherein a compound library is subjected to a first binding assay with a protein of SEQ ID NO.1 and/or SEQ ID NO.2, and to a second binding assay with the mutant R186A or R189A of the protein of SEQ ID NO.1 and/or SEQ ID NO.2, and selecting the compound(s) the inhibitory action in the first binding assay is larger than the one in the second binding assay. The present invention also provides a method for providing a compound that binds to the region composed of amino acids at positions 170 - 199 of SEQ ID NO.1 and/or SEQ ID NO.2, wherein a compound library is subjected to a first binding assay with a protein of SEQ ID NO.1 and/or SEQ ID NO.2, and to a second binding assay with the mutant R186A or R189A of the protein of SEQ ID NO.1 and/or SEQ ID NO.2, and selecting the compound(s) whose inhibitory action in the first binding assay is larger than the one in the second binding assay, and synthesizing the compound or ordering the compound from a provider.

The following examples are intended for the sole purpose of illustrating the present invention.

### Examples

### Example 1: Discovery of novel binding site

The national for considering the specific region composed of amino acids at positions 170 - 199, herein disclosed as SEQ ID NO.4 for Mitogen-activated protein kinase 14 and SEQ ID NO.5 for Mitogen-activated protein kinase 11 is based in the analysis of the crystal structure of the p38a available from the Protein Data Bank (PDB entry 2OZA). As a proof of concept, Peptide 1 comprising SEQ ID NO.3 belonging to an amino acidic sequence of protein Kinase MK2 was selected for binding to the site.

To analyze the effect of the inhibitory peptides, we performed in vitro kinase assays (IN VITRO PHOSPHORYLATION ASSAY: ADP Quest Method). Purified active p38alpha (25nM; Invitrogen) was preincubated with the peptides 1 (seven aminoacids) and 2 (six aminoacids) for 10 minutes at 30°C at concentrations ranging from 4 to 1000mM in a final volume of 40ul of kinase buffer (Hepes 15mM pH 7.4, NaCl 20mM, EGTA 1mM, Tween 20 0,02%, MGCl₂ 10mM, gamma-globulins 0,1%). After that, GST-MEF2A (300nM) and ATP (100uM) were added and incubated 30 minutes at 30°C for the kinase reaction. Phosphorylation was measured as ADP production and ADP was detected using the ADP Quest assay kit (DiscoveRx Corp) following manufacturers instructions. Resultant fluorescence was read with BMG Fluostar microplate reader.

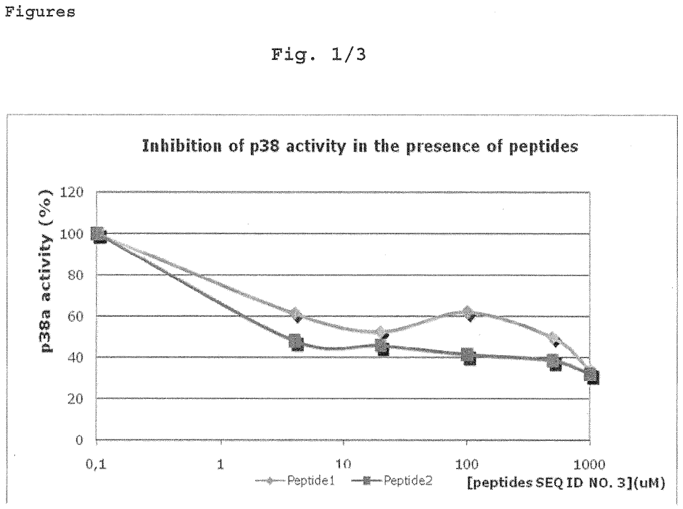

The results are shown in Figure 1.

### Example 2 : Identification of compounds fulfilling pharmacophore requirements.

In order to identify hits against the novel binding site where the peptides with SEQ ID NO. 3 bind (as described above), an *in silico* screening of commercially available compounds, such as database ZINC (http://zinc.docking.org), was performed using the pharmacophore defined previously as identification criterion.

The process for assessing if a new compound fulfills pharmacophore requirements is done following a hierarchical procedure:
First, the different chemical moieties are identified. For a compound to be considered for a further study, it should exhibit at least four of the requirements of the pharmacophore. Then, the distances between moieties in the molecule are compared with those of the pharmacophore. Finally, if the distances fulfill pharmacophore requirements, molecules are superimposed with the pharmacophore through geometrical superimposition. The root-mean-square-deviation, between the query features and their matching ligand annotation points, is then used to assess the degree of fulfillment of the pharmacophore.

Hits identified are screening further to assess their drug-like properties using Lipinski's rule of five. The final set of compounds is submitted to a molecular docking procedure. In order to reduce the dimension of the final set, compounds are clustered using the Jarvis-Patrick non-hierarchic method using a 3-point pharmacophore fingerprints as descriptors.

This procedure was followed to identify Compounds 1 to 11 which were purchased and tested in a p38 binding assay.

### Example 3: Binding assay

### 3.1. Materials and Methods

To examine the inhibition of the Compounds 1 to 11, in vitro kinase assays were performed. Purified recombinant activated p38alpha (10nM) (ProQinase) was preincubated 10 minutes at 30°C with the compound of interest at concentrations between 1 and 100µM in duplicate in a final volume of 30ul of kinase buffer (Hepes 60mM pH 7.5, MgCl2 3mM, MnCl2 3mM, Sodium Orthovanadate 3µM, DTT 1.2mM). After preincubation, peptide substrate (SignalChem #P03-58) and ATP were added to a final concentration of 10 µM and 100 µM respectively, and then incubated 40 minutes at 30°C for the kinase reaction. Phosphorylation was analyzed with ADP-Glo™ (Promega #V9101) and emitted luminescence was measured with BMG Fluostar microplate reader.

For p38 cascade kinase reaction, activated p38alpha was substituted by inactive p38alpha (10nM) (Invitrogen #PV3305) and preincubated 10 minutes at 30°C with the compound of interest in kinase buffer. After preincubation, MKK6 (ProQinase), ATF2 (ProQinase) and ATP were added to a final concentration of 10nM, 5µM and 10µM respectively, then incubated 40 minutes at 30°C and analyzed with ADP-Glo™.

### 3.2. VENERATION OF P38αR186A AND P38αR189A MUTANTS:

### Replacement of specific residues arginine 186 and 189 of p38α

(pGEX4T-1-p38α) for alanine was performed with the QuikChange Lightining Site-Directed Mutagenesis Kit (Agilent Technologies). The following oligonucleotides were used for the arginine 186 to alanine substitution: forward primer: 5'- SEQ ID NO 7 -3'; reverse primer: 5'- SEQ ID NO 8 -3'. Oligonucleotides used for the arginine 189 to alanine substitution were: forward primer: 5'- SEQ ID NO 9 -3' ; reverse primer: 5'- SEQ ID NO 10 -3'. Mutations were validated by sequencing (Genomic Unit, Parque Científico de Madrid). E. coli XL10-Gold ultracompentent cells (Agilent Technologies) were transformed with the vector plasmids containing p38αWT and mutants. For the expression of recombinant p38α transformed cells were cultured overnight at 37°C in 50ml of fresh LB medium containing ampicillin (100µg/ml). After that, culture was diluted in fresh LB to A595= 0.4 and grown for approximately 1h at 37°C until exponential growth and then induced with Isopropyl-β-D-thiogalactopyranoside (IPTG) 1mM for 5h. The cells were collected by centrifugation and cell pellet were resuspended in 50ml of cold lysis buffer: PBS pH 7.4, EDTA 10mM, PMSF 1mM, Benzamidine 0.2mM, DTT 5mM, Triton X-100 1% and Lysozyme 1mg/ml. The soluble and insoluble fractions were separated by centrifugation (16,000g for 20 min). The soluble fraction was incubated for 2 hours with 1ml of pre-equilibrated Glutathione Sepharose 4B (GE Healthcare) and the resin was extensively washed in a column with 200ml of binding buffer: PBS, PMSF 1mM and benzamidine 0.2mM. Proteins were eluted with 1ml of Tris-HCl 50mM pH 8.0 and reduced glutathione 20mM. 20% glycerol was added to purified proteins and stored at -80°C. Protein concentration and purity was assessed by SDS-PAGE. Kinase activity was measured by ADP-glo kit using MKK6 as activator and ATF2 as substrate. Activity recorded for p38αR186A and p38αR189A were 15,5% and 4,8%, respectively (n=4) related to wild type protein.

### 3.3. Results of the binding assay

### 1. Inhibitory assays over p38 cascade using different compounds predicted by the docking algorithm

| | p38a/ATF-2 |
|---|---|
| Compound 2*(10µM) | 2% |
| Compound 3*(100 µM) | 17% |
| Compound 4*(10 µM) | 8% |
| Compound 5*(10 µM) | 28% |
| Compound 6*(10 µM) | 107% |
| Compound 7*(10 µM) | 16% |
| Compound 8*(100 µM) | 20% |
| Compound 9*(10 µM) | 9% |
| Compound 10*(10 µM) | 43% |
| Compound 11*(10 µM) | 1% |

| | |
|---|---|
| *Reference Compounds | |

### 2. Inhibitory assays over four different MAP Kinase proteins: dose response achieved with Compound 1

| | p38alpha/ATF-2 | p38beta/ATF-2 |
|---|---|---|
| Compound 1 (100µM) | 101% | 100% |
| Compound 1 (10µM) | 88% | 58% |
| SB (10µM) | 104% | 102% |

### 3. ATP-site competitive assay: Compound 1 maintains inhibition to the same level upon ATP increase but not SB (n=4) in cascade p38alpha/ATF-2

| | ATP 10µM | ATP 100µM |
|---|---|---|
| Compound 1 (10µM) | 94% | 92% |
| SB (1µM) | 81% | 67% |

### 4. Assay of compounds in site-mutated p38 over phosphorylation cascade: the compounds decrease inhibitory capacity but SB does not.

| 10uM | p38alpha wt | R186A | R189A |
|---|---|---|---|
| Compound 1 | 64% | 20% | 25% |
| Compound 12* | 81% | 39% | 14% |
| Compound 13 | 34% | 22% | 14% |
| Compound 14 | 31% | 18% | 17% |
| Compound 15 | 61% | 28% | 20% |
| Compound 16 | 32% | 15% | 17% |
| Compound 17 | 56% | 19% | 13% |
| Compound 18 | 62% | 37% | 33% |
| Compound 19 | 71% | 35% | 32% |
| Compound 20 | 71% | 38% | 22% |
| Compound 21 | 83% | 48% | 38% |
| Compound 22* | 31% | 16% | 6% |
| Compound 23 | 90% | 48% | 26% |
| Compound 24* | 66% | 43% | 39% |
| Compound 25 | 59% | 14% | 30% |
| Compound 25* | 73% | 40% | 28% |
| Compound 27* | 88% | 72% | 47% |
| Compound 28 | 86% | 41% | 27% |
| Compound 29 | 23% | 21% | 6% |
| Compound 30 | 25% | 19% | 9% |
| Compound 31 | 84% | 35% | 27% |
| Compound 32* | 23% | 17% | 9% |
| Compound 33 | 47% | 23% | 19% |
| SB (1uM) | 60% | 80% | 76% |

| | | | |
|---|---|---|---|
| *Reference Compounds SB: An ATP-site competitive inhibitor of p38. It is the standard compound to be used as positive Control. | | | |

### Example 4: Cell-based experiments: results, materials and methods

### 4.1. Fibroblast-like synoviocytes (FLS)

### 4.1.1. Materials and Methods: Fibroblast-like synoviocytes (FLS)

Fibroblast-like synoviocytes (FLS) from patients with rheumatoid arthritis (RA) were obtained at the time of synovectomy or joint replacement. All RA patients fulfilled the American College of Rheumatology 1997 criteria for the diagnosis of RA (Arnett FC y cols. 1988 Arthritis Rheum 31(3):315-24).

Synovial tissue was minced and incubated with 10 µg/ml collagenase in serum-free Dulbecco modified Eagle medium (DMEM; Gibco Invitrogen, Barcelona, Spain) for 3 h at 37°C. After digestion, FLS were filtered through a nylon cell strainer (BD Falcon, Franklin Lakes,USA), washed extensively, and cultured in DMEM supplemented with 10% v/v fetal calf serum, 1% penicillin-streptomycin, and 1% L-glutamine (all reagents from PAA, Laboratories GmbH) in a humidified 5% CO2 atmosphere. Adherent cells at 80-90 % confluence were trypsinised and diluted at a split ratio of 1:3. FLS from 4 patients and between passages 3 to 8 were used for all experiments.

Enzyme-Linked Immunosorbent Assay (ELISA): RA FLS (1x104cells/well) were cultured in DMEM 1% FCS in 96 well plates and treated with p-p38 inhibitors for 1h. After, they were stimulated with TNF-α (10 ng/ml, Sigma Aldrich) and both treatments were present in the culture throughout the experiment. The supernatants were harvested at 18h after treatment and assayed for IL6, IL8/CXCL8 and monocyte chemoattractant protein (MCP)-1/CCL2 by ELISA according to the manufacturer's instructions (BD Bioscience, San Jose, California, USA).

### 4.1.2 Results: Fibroblast-like synoviocytes (FLS)

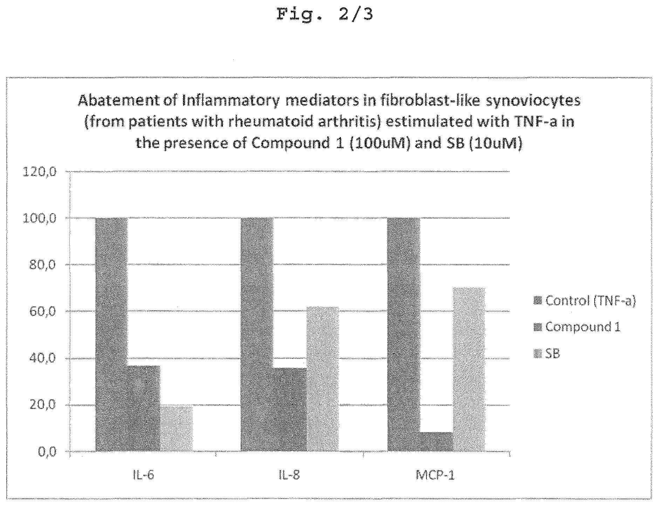

Endpoint: abetment of inflammatory mediators (IL6, IL8 and MCP-1) in FLS stimulated with TNF-alpha in the presence of compounds 1 and SB (positive control). Please also refer to Fig. 2.

| IL-6 | | | | | | |
|---|---|---|---|---|---|---|
| | **SH203(%)** | **R8(%)** | **R 6**(%) | **R 2**(%) | **Media** | **ESM** |
| **CONTROL** | | | | | | |
| **TNF-α** | 100 | 100 | 100 | 100 | 100,0 | 0,0 |
| **100µM Comp**.**1** | 101,4 | 23,5 | 8,1 | 13,8 | 36,7 | 21,8 |
| **SB 10µM** | 57,0 | 17,1 | 35,4 | 11,3 | 19,6 | 4,7 |
| | | | | | | |

| **IL-8** | | | | | | |
|---|---|---|---|---|---|---|
| | **SH203**(%) | R8(%) | **R 6**(%) | **R 2(%)** | **Media** | **ESM** |
| **CONTROL** | | | | | | |
| **TNF-α** | 100 | 100 | 100 | 100 | 100,0 | 0,0 |
| **100µM Comp**.**1** | 43,8 | 67,4 | 4,8 | 27.3 | 35,8 | 13,2 |
| **SB 10µM** | 64,4 | 83,9 | 54,1 | 47,8 | 62,1 | 272,7 |
| | | | | | | |

| **MCP**-**1** | | | | | | |
|---|---|---|---|---|---|---|
| | **SH203(%)** | **R 8(%)** | **R 6(%)** | **R 2(%)** | **Media** | **ESM** |
| **CONTROL** | | | | | | |
| **TNF-α** | 100 | 100 | 100 | 100 | 100,0 | 0,0 |
| **100µM Comp**.**1** | 8,8 | 22,5 | 0,0 | 2,1 | 8,4 | 5,1 |
| **SB 10µM** | 77,6 | 76,2 | 63,5 | 61,9 | 70,2 | 78,3 |

Other compounds tested in synoviocytes exerted the following inhibition capacity
% of inflammatory mediators production in the presence of compounds

| [100uM] | IL6 | IL8 | MCP-1 |
|---|---|---|---|
| TNFa | 100% | 100% | 100% |
| TNFa + Compound 8* | 66,5% | 83,4% | 75,4% |
| TNFa + Compound 9* | 85,4% | 83,2% | 62,5% |
| TNFa + Compound 32* | 52,4% | 34,8% | 22,7% |

| | | | |
|---|---|---|---|
| *Reference Compounds | | | |

### 4.2. Chondrocytes and macrophages

### 4.2.1 Materials & Methods: Chondrocytes and macrophages

Cell viability was examined using a colorimetric assay based on the MTT labeling reagent. Briefly, Cells (8 x 103/well) were seeded in 96-well plates. Assays were performed according to the instructions and protocol provided by the manufacturer (Sigma-Aldrich). After complete adhesion, cells were cultures in serum free conditions (overnight) in order to synchronize cell cycle. Next, cells were incubated with the pertinent drug for 24 or 48 hours with (0.1-50 µM) alone or in combination with 5% FBS medium for 24-48 hours at 37°C. After that, cells were incubated with 10 µl of MTT (5 mg/ml) for 4 hours at 37°C. Then, after dissolving the formazan salt, the spectrophotometric absorbance was measured using a microtiter enzyme-linked immunosorbent assay reader at 550 nm (Multiskan EX; Thermo Labsystems). Data are expressed as % of vitality over unstimulated control. Cell lines used: ATDC5 mouse chondrogenic and J774A1 murine macrophages.

Cell treatments and nitrite assay: ATDC5 cells, as well J774 murine macrophages, with viability greater than 95% evaluated by Trypan blue exclusion method, were cultured in standard conditions in 24-well plates. After 12 h of starvation in serum-free medium, cells were stimulated for 24-48 h with LPS 250nM, alone or in combination with the drugs. Nitrite accumulation was measured in the culture medium by Griess reaction. Briefly, 100 µl of cell culture medium was mixed with 100 µl of Griess reagent [equal volumes of 1% (wt/vol) sulfanilamide in 5% (vol/vol) phosphoric acid and 0.1% (wt/vol) naphtylethylenediamine-HCl], incubated at room temperature for 10 min, and then the absorbance at 550 nm was measured in a microplate reader (Titertek-Multisca, Labsystem). Fresh culture medium was used as blank in all the experiments. The amount of nitrite in the samples (in micromolar units) was calculated from a sodium nitrite standard curve freshly prepared in culture medium.

### 4.2.2. Results: Chondrocytes and macrophages

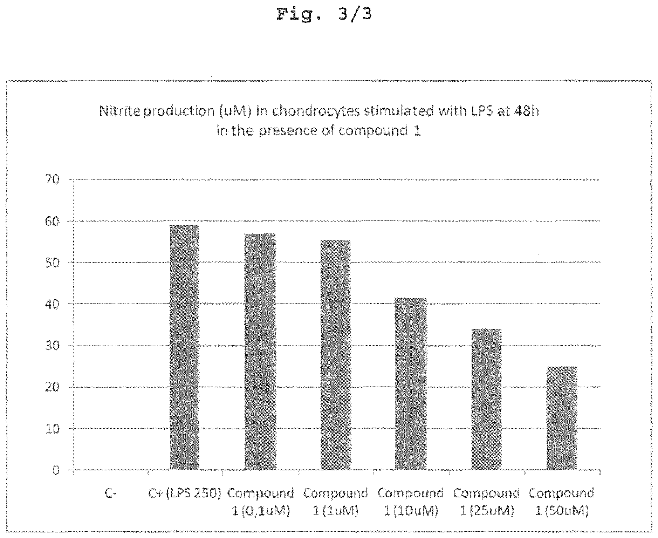

Please refer to Figure 3.

### 4.3. Human Monocytic Cell Line

### 4.3.1. Materials & Methods: Human Monocytic Cell Line

Inhibition of TNF-α Secretion by a Human Monocytic Cell Line, THP-1.

THP-1 cells, growing in log phase, were collected by centrifugation and resuspended in RPMI 1640 (Sigma Aldrich), to a final cell concentration of 2 x 106 cells/ml. Cells were plated into 24-well plates (BD Biosciences). Dilutions of compounds in dimethyl sulfoxide (DMSO) were added to the culture to a final concentration M. The final DMSO concentration was 0,5%. The cells ranging from 0,1 to 50 suspensions were preincubated with compounds for 1h at 37°C in a 5% CO2 humidified atmosphere before the addition of LPS (Sigma Aldrich, L2654) to a final concentration of 2 ug/ml. After that, cells were incubated for 3h followed by centrifugation to pellet cells. Cell supernatants were stored at 4°C until Human analysis for TNF-α content. TNF-α levels were determined by ELISA (TNF Biotrak, GE Healthcare) following the manufacturer's directions. The percentage inhibition was calculated for each compound concentration tested, and the IC50 was calculated for each compound.

### 4.3.2. Results: Abetment of TNF alpha production in monocytes and survival of monocytes with LPS in the presence of compounds 1 and 12 to 31: (see table below)

| | TNF abetment | | | Cell survival | | |
|---|---|---|---|---|---|---|
| | 0,1uM | 1uM | 10uM | 0,1uM | 1uM | 10uM |
| Compound 1 | | 9% | 40% | | 96% | 93% |
| Compound 12* | | -6% | -16% | | 97% | 99% |
| Compound 13 | | 13% | 32% | | 93% | 86% |
| Compound 14 | | | 17% | | | 81% |
| Compound 15 | | 17% | 32% | | 92% | 97% |
| Compound 16 | 16% | 26% | 35% | 88% | 95% | 93% |
| Compound 17 | 21% | 34% | 69% | 99% | 98% | 97% |
| Compound 18 | 1% | 34% | 56% | 90% | 83% | 79% |
| Compound 19 | -47% | -21% | -10% | 103% | 97% | 93% |
| Compound 20 | -9% | 55% | 36% | 101% | 96% | 81% |
| Compound 21 | -15% | 18% | 20% | 98% | 95% | 94% |
| Compound 22* | -2% | 17% | 10% | 97% | 94% | 99% |
| Compound 23 | 26% | 41% | 60% | 96% | 95% | 88% |
| Compound 24* | -23% | 12% | 67% | 104% | 98% | 105% |
| Compound 25 | 12% | 61% | 102% | 88% | 61% | 42% |
| Compound 26* | 27% | 30% | 8% | 86% | 87% | 83% |
| Compound 27* | 28% | 58% | 76% | 103% | 99% | 72% |
| Compound 28 | 17% | 24% | 36% | 88% | 87% | 88% |
| Compound 29 | 7% | 11% | 44% | 103% | 103% | 92% |
| Compound 30 | 12% | 16% | 41% | 99% | 97% | 93% |
| Compound 31 | 20% | 43% | 60% | 101% | 100% | 92% |
| Compound 32* | | | 21% | | | 99% |
| Compound 33 | 21% | 46% | 62% | 100% | 99% | 97% |

| | | | | | | |
|---|---|---|---|---|---|---|
| *Reference Compounds | | | | | | |

## Claims

1. A compound for use in a method for treatment of p38 mediated diseases or disorders, wherein
the compound binds to the region composed of amino acids at positions 170 - 199 of SEQ ID NO.1 of MAPK14 (p38alpha) and/or SEQ ID NO.2 of MAPK11 (p38beta); and
wherein the compound:
is a peptide of up to 20 amino acids in length and which comprises a hexa-or heptapeptide having anyone of SEQ ID NO 3 or SEQ ID NO 6; or
is a heterocylic compound of the following general formula: 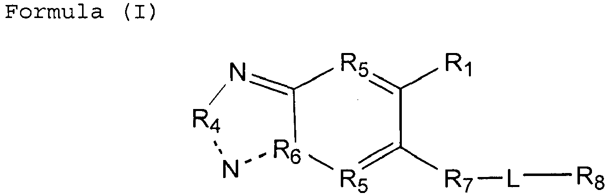 wherein
R1 is NR2R3, or OR2;
R2 is C5-C7-aryl or C5-C7-heteroaryl;
wherein the C5-C7-aryl or C5-C7-heteroaryl, group is substituted with one or more of a -OR10, -SR10, -NHCOR10, -CONHR10, -COR10,-COOR10 , -OCOR10, -NR10R11, -SO2R10, -SO2NR10R11 -CF3, -OCF3 or-CN, wherein R10 and R11 are independently of each other hydrogen, C1-C4-alkyl, C1-C4-alkenyl or C1-C4-alkynyl; and
wherein if R1 is NR2R3, the C5-C7-aryl or C5-C7-heteroaryl, can be connected to the N via a single bond or NH;
**R3** is hydrogen or a C1-C4-alkyl group;
**R4---N---R6** is N=N-N or O-N=C;
**R5** is independently of each other N or CR11; wherein R11 is hydrogen, OH, or SH;
**R7** is an N-containing saturated or unsaturated C3-C7 heterocycloalkyl;
L is a linker L, preferably a single covalent bond (so that R7 is linked directly to R8) or a C1 to C3 alkylen group, wherein one of the CH2 groups may be replaced by O, NH, or S;
**R8** is C5-C10-aryl or C5-C10-heteroaryl, which may be substituted at 4 position with a halogen, C1-C4 alky group, -OR12 or -SR12; wherein R12 is a C1 to C4 alkyl group;
or a pharmaceutically acceptable salt, ester, and amide thereof; and
wherein the p38 mediated diseases or disorders are selected from acute and/or chronic inflammatory diseases, macular degeneration, chronic pulmonary inflammatory disease and Alzheimer's disease.

2. The compound for use in a method for treatment according to claim 1, wherein the compound
a) fulfils a pharmacophore structure wherein five points are defined as follows:
Point A: a hydrophobic/aromatic moiety;
Point B: a hydrophobic/aromatic moiety;
Point C: a hydrogen acceptor point;
Point D: a hydrogen acceptor point; and
Optional: Point E: a hydrogen donor,
and wherein the spatial relation between the pharmacophore points are specified by spheres of different radii (r) centered at their Cartesian coordinates: point A (7.99, 34.86 40.16), r=1.5; Point B (12.16; 36.69; 37.15), r= 1.6 ; Point C (7.03, 33.05, 40.27), r= 1.2; Point D (9.30, 37.74, 46.62), r=1.5; Point E (12.72, 36.93, 35.072), r= 1.6; and
b) Binds to the region composed of amino acids at positions 170 - 199 of SEQ ID NO.1 of MAPK14 (p38alpha) and/or SEQ ID NO.2 of MAPK11 (p38beta).

3. The compound for use in a method for treatment according to claim 1 , wherein the compound has an inhibitory effect on the protein of SEQ ID NO.1 and/or of SEQ ID NO.2, but to a lesser extent on the mutant R186A or R189A of the protein of SEQ ID NO.1 and/or of SEQ ID NO.2.

4. The compound for use in a method for treatment according to anyone of claims 1 to 3, for use in a method for treatment or prevention of inflammatory diseases or disorders.

5. The compound for use in a method for treatment according to anyone of claims 1 to 3, for use in a method for treatment or prevention of rheumatoid arthritis, osteoarthritis, psoriatic arthritis, inflammatory pain and musculoskeletal system inflammation.

6. The compound for use in a method for treatment according to one or more of the precedent claims, wherein the compound has an IC50 of 0.01 nM to 100 µM for inhibiting the MAPK p38 protein activity.

7. The compound for use in a method for treatment according to claim 6, wherein the compound has an IC50 of 0.1 nM to 10 µM for inhibiting the MAPK p38 protein activity.

8. The compound for use in a method for treatment according to claim 1, wherein
**R1** is NR2R3;
**R2** is C6-aryl or C5-C6-heteroaryl, which is substituted with one of a -OR10, -SR10, -NHCOR10, -CONHR10, -COR10, -COOR10 , -OCOR10, -NR10R11, -SO2R10, -SO2NR10R11, -CF3, -OCF3 or -CN, wherein R10 and R11 are independently of each other hydrogen, C1-C4-alkyl, C1-C4-alkenyl or C1-C4-alkynyl;
wherein C6-aryl, or C5-C6-heteroaryl can be connected to the N via a single bond or NH;
**R3** is H;
**R4---N---R6** is N=N-N or O-N=C;
**R5** is independently of each other N or CR11, wherein R11 is hydrogen;
**R7** is an N-containing saturated or unsaturated C6 heterocycloalkyl;
L is a single covalent bond (so that R7 is linked directly to R8) or a C1 to C3 alkylen group, wherein one of the CH2 groups may be replaced by O, NH, S or CO;
**R8** is C6-C10-aryl or C6-C10-heteroaryl.

9. The compound for use in a method for treatment according to claim 1 or 8, wherein the compound has the following Formula: 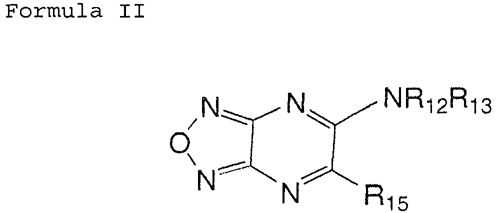 and wherein
R12 is hydrogen, or a C1 to C4 alkyl group;
R13 is a ring structure,
the ring structure being an aryl ring which is substituted at positions 3 or 4 with, CF3, OCF3, OR14, NHCOR14, COR14, CONHR14 or COOR14, wherein R14 is a C1 to C8 alkyl group;
or an O-, N- and/or S-containing heterocyclic, saturated or non-saturated ring with 5 to 7 ring members which is substituted at 3 or 4 position with a halogen atom, CF3, or OCF3, OR14, NHCOR14, COR14, or COOR14, wherein R14 is a C1 to C8 alkyl group, and which may be substituted with a halogen atom, CF3, OCF3, a C1 to C4 alkyl group, OR14, NHCOR14,COR14,or COOR14, wherein R14 is H or a C1 to C8 alkyl group at the remaining positions;
wherein the ring structure or the O-, N- and/or S-containing heterocyclic can be connected to the N via a single bond or NH;
R15 is an N-containing heterocycle, with the N binding to the oxazolidinone ring structure, which is linked, via a linker, to either
a C6-C10-aryl or a phenyl ring which might be substituted at 4 position with OR14, NHCOR14, COR14, COOR14, wherein R14 is a C1 to C8 alkyl group, a halogen atom, CF3, or OCF3, and which may be substituted with a C1 to C4 alkyl group, a halogen atom, OR14, NHCOR14, COR14, COOR14, wherein R14 is a C1 to C8 alkyl group a halogen atom CF3, or OCF3 at the remaining positions;
or an O-, N- and/or S-containing heterocyclic, saturated or non-saturated ring with 5 to 7 ring members, and which may be substituted with a C1 to C4 alkyl group, OR14, NHCOR14, COR14, COOR14, wherein R14 is a C1 to C8 alkyl group a halogen atom CF3, or OCF3 at the remaining positions;
or a pharmaceutically acceptable salt, ester, and amide thereof.

10. Pharmaceutical composition comprising anyone of the following compounds: 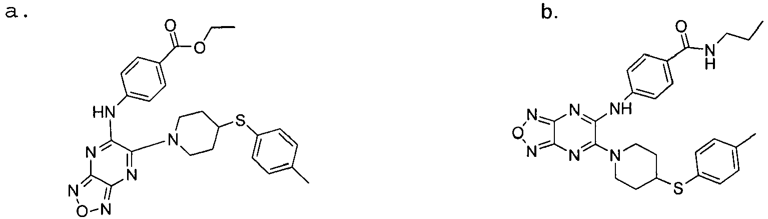 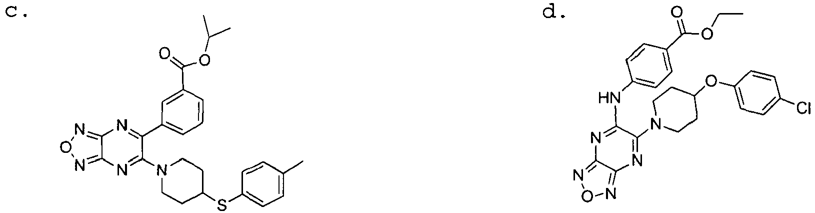 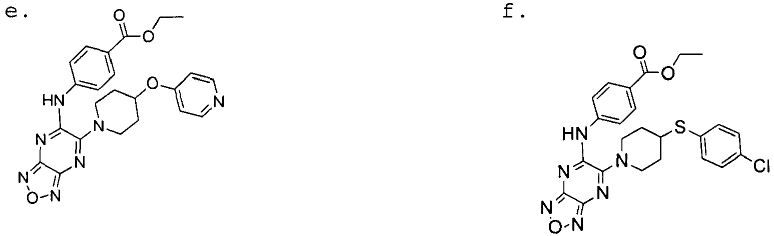 or a pharmaceutically acceptable salt, ester, and amide thereof; and
a pharmaceutically acceptable excipient.

11. Method for screening a compound that binds to the region composed of amino acids at positions 170 - 199 of SEQ ID NO.1 and/or SEQ ID NO.2, wherein a compound library is subjected to a first binding assay with a protein of SEQ ID NO.1 and/or SEQ ID NO.2, and to a second binding assay with the mutant R186A or R189A of the protein of SEQ ID NO.1 and/or SEQ ID NO.2, and selecting the compound(s) the inhibitory action in the first binding assay is larger than the one in the second binding assay.

12. Method for providing a compound that binds to the region composed of amino acids at positions 170 - 199 of SEQ ID NO.1 and/or SEQ ID NO.2, wherein a compound library is subjected to a first binding assay with a protein of SEQ ID NO.1 and/or SEQ ID NO.2, and to a second binding assay with the mutant R186A or R189A of the protein of SEQ ID NO.1 and/or SEQ ID NO.2, and selecting the compound(s) whose inhibitory action in the first binding assay is larger than the one in the second binding assay, and synthesizing the compound or ordering the compound from a provider.

## Patentansprüche

1. Verbindung zur Verwendung in einem Verfahren zur Behandlung von p38-vermittelten Erkrankungen oder Störungen, wobei
die Verbindung an die Region bindet, die aus Aminosäuren an Positionen 170-199 von SEQ ID NO: 1 von MAPK14 (p38alpha) und/oder SEQ ID NO: 2 von MAPK11 (p38beta) zusammengesetzt ist; und
wobei die Verbindung
ein Peptid von bis zu 20 Aminosäuren Länge ist und welches ein Hexa- oder Heptapeptid umfasst, welches irgendeine von SEQ ID NO: 3 oder SEQ ID NO: 6 hat; oder
eine heterozyklische Verbindung der folgenden allgemeinen Formel ist: 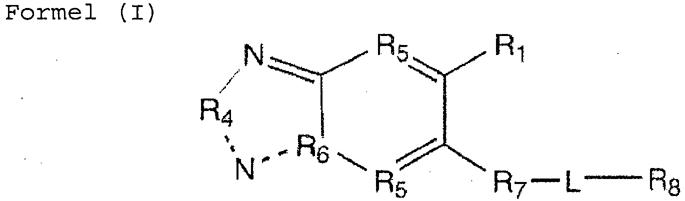 worin
R1 NR2R3 oder OR2 ist;
R2 C5-C7-Aryl oder C5-C7-Heteroaryl ist;
wobei die C5-C7-Aryl- oder C5-C7-Heteroarylgruppe mit einem oder mehreren aus einem -OR10, -SR10, -NHCOR10, -CONHR10, -COR10, -COOR10, -OCOR10, -NR10R11, -SO2R10, -SO2NR10R11, -CF3, -OCF3 oder -CN substituiert ist, worin R10 und R11 unabhängig voneinander Wasserstoff, C1-C4-Alkyl, C1-C4-Alkenyl oder C1-C4-Alkynyl sind; und
wobei, wenn R1 NR2R3 ist, das C5-C7-Aryl oder C5-C7-Heteroaryl mit dem N über eine Einfachbindung oder NH verbunden sein kann;
R3 Wasserstoff oder eine C1-C4-Alkylgruppe ist;
R4---N---R6 N=N-N oder O-N=C ist;
R5 unabhängig voneinander N oder CR11 ist; worin R11 Wasserstoff, OH oder SH ist;
R7 ein N-enthaltendes gesättigtes oder ungesättigtes C3-C7-Heterocycloalkyl ist;
L ein Linker L ist, bevorzugt eine einfache Kovalenzbindung (so dass R7 direkt mit R8 verknüpft ist) oder eine C1- bis C3-Alkylengruppe, worin eine der CH2-Gruppen durch O, NH oder S ersetzt werden kann;
R8 C5-C10-Aryl oder C5-C10-Heteroaryl ist, welches an der 4-Position mit einem Halogen, einer C1-C4-Alkylgruppe, -OR12 oder -SR12, worin R12 eine C1- bis C4-Alkylgruppe ist, substituiert sein kann;
oder ein pharmazeutisch akzeptable(s/r) Salz, Ester und Amid davon; und
wobei die p38-vermittelten Erkrankungen oder Störungen aus akuten und/oder chronischen entzündlichen Erkrankungen, Makuladegeneration, chronischer Lungenentzündungserkrankung und Alzheimer'scher Erkrankung ausgewählt sind.

2. Verbindung zur Verwendung in einem Verfahren zur Behandlung gemäß Anspruch 1, wobei die Verbindung
a) eine Pharmakophor-Struktur erfüllt, wobei fünf Punkte wie folgt definiert sind:
Punkt A: ein hydrophober/aromatischer Rest;
Punkt B: ein hydrophober/aromatischer Rest;
Punkt C: ein Wasserstoffakzeptorpunkt;
Punkt D: ein Wasserstoffakzeptorpunkt; und
Optional: Punkt E: ein Wasserstoffdonor,
und wobei das räumliche Verhältnis zwischen den Pharmakophor-Punkten durch Kugeln unterschiedlicher Radien (r) spezifiziert wird, die an ihren kartesischen Koordinaten zentriert sind: Punkt A (7,99, 34,86 40,16), r = 1,5; Punkt B (12,16; 36,69; 37,15), r = 1,6; Punkt C (7,03, 33,05, 40,27), r = 1,2; Punkt D (9,30, 37,74, 46,62), r = 1,5); Punkt E (12,72, 36,93, 35,072), r = 1,6; und
b) an die Region bindet, die aus Aminosäuren an Positionen 170-199 von SEQ ID NO: 1 von MAPK14 (p38alpha) und/oder SEQ ID NO: 2 von MAPK11 (p38beta) zusammengesetzt ist.

3. Verbindung zur Verwendung in einem Verfahren zur Behandlung gemäß Anspruch 1, wobei die Verbindung eine inhibitorische Wirkung auf das Protein von SEQ ID NO: 1 und/oder SEQ ID NO: 2 hat, jedoch in geringerem Umfang auf die Mutante R186A oder R189A des Proteins von SEQ ID NO: 1 und/oder von SEQ ID NO: 2.

4. Verbindung zur Verwendung in einem Verfahren zur Behandlung gemäß irgendeinem der Ansprüche 1 bis 3, zur Verwendung in einem Verfahren zur Behandlung oder Prävention entzündlicher Erkrankungen oder Störungen.

5. Verbindung zur Verwendung in einem Verfahren zur Behandlung gemäß irgendeinem der Ansprüche 1 bis 3, zur Verwendung in einem Verfahren zur Behandlung oder Prävention von rheumatoider Arthritis, Osteoarthritis, psoriatischer Arthritis, Entzündungsschmerz und Entzündung des Muskel-Skelett-Systems.

6. Verbindung zur Verwendung in einem Verfahren zur Behandlung gemäß einem oder mehreren der vorangegangenen Ansprüche, wobei die Verbindung eine IC50 von 0,01 nM bis 100 µM zur Inhibition der MAPK-p38-Proteinaktivität hat.

7. Verbindung zur Verwendung in einem Verfahren zur Behandlung gemäß Anspruch 6, wobei die Verbindung eine IC50 von 0,1 nM bis 10 µM zur Inhibition der MAPK-p38-Proteinaktivität hat.

8. Verbindung zur Verwendung in einem Verfahren zur Behandlung gemäß Anspruch 1, wobei
R1 NR2R3 ist;
R2 C6-Aryl oder C5-C6-Heteroaryl ist, welches mit -OR10, -SR10, -NHCOR10, -CONHR10, -COR10, -COOR10, -OCOR10, -NR10R11, -SO2R10, -SO2NR10R11, -CF3, -OCF3 oder -CN substituiert ist, worin R10 und R11 unabhängig voneinander Wasserstoff, C1-C4-Alkyl, C1-C4-Alkenyl oder C1-C4-Alkynyl sind;
wobei C6-Aryl oder C5-C6-Heteroaryl mit dem N über eine Einfachbindung oder NH verbunden sein kann;
R3 H ist;
R4---N---R6 N=N-N oder O-N=C ist;
R5 unabhängig voneinander N oder CR11 ist, worin R11 Wasserstoff ist;
R7 ein N-enthaltendes gesättigtes oder ungesättigtes C6-Heterocycloalkyl ist;
L eine einfache Kovalenzbindung (so dass R7 direkt mit R8 verknüpft ist) oder eine C1- bis C3-Alkylengruppe ist, wobei eine der CH2-Gruppen durch O, NH, S oder CO ersetzt werden kann;
R8 C6-C10-Aryl oder C6-C10-Heteroaryl ist.

9. Verbindung zur Verwendung in einem Verfahren zur Behandlung gemäß Anspruch 1 oder 8, wobei die Verbindung die folgende Formel hat: 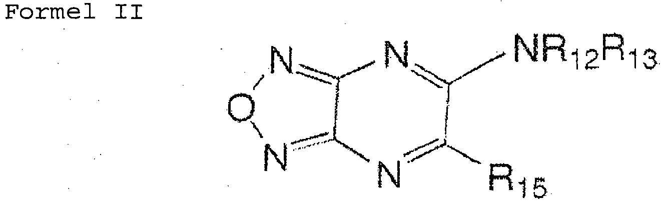 und wobei
R12 Wasserstoff oder eine C1- bis C4-Alkylgruppe ist;
R13 eine Ringstruktur ist,
wobei die Ringstruktur ein Arylring ist, welcher an den Positionen 3 oder 4 mit CF3, OCF3, OR14, NHCOR14, COR14, CONHR14 oder COOR14, worin R14 eine C1- bis C8-Alkylgruppe ist, substituiert ist;
oder ein O-, N- und/oder S-enthaltender heterozyklischer, gesättigter oder ungesättigter Ring mit 5 bis 7 Gliedern, der an der 3- oder 4-Position mit einem Halogenatom, CF3 oder OCF3, OR14, NHCOR14, COR14 oder COOR14, worin R14 eine C1- bis C8-Alkylgruppe ist, substituiert ist, und welcher an den verbleibenden Positionen mit einem Halogenatom, CF3, OCF3, einer C1-bis C4-Alkylgruppe, OR14, NHCOR14, COR14 oder COOR14, worin R14 H oder eine C1- bis C8-Alkylgruppe ist, substituiert sein kann;
wobei die Ringstruktur oder der O-, N- und/oder S-enthaltende Heterozyklus mit dem N über eine Einfachbindung oder NH verbunden sein kann;
R15 ein N-enthaltender Heterozyklus ist, wobei N an die Oxazolidinon-Ringstuktur bindet, welche über einen Linker verknüpft ist mit entweder
einem C6-C10-Aryl oder einem Phenylring, welcher an der 4-Position mit OR14, NHCOR14, COR14, COOR14, worin R14 eine C1- bis C8-Alkylgruppe, ein Halogenatom, CF3 oder OCF3 ist, substituiert sein kann, und welcher an den verbleibenden Positionen mit einer C1- bis C4-Alkylgruppe, einem Halogenatom, OR14, NHCOR14, COR14, COOR14, worin R14 eine C1- bis C8-Alkylgruppe, ein Halogenatom, CF3 oder OCF3 ist, substituiert sein kann;
oder einem O-, N- und/oder S-enthaltenden heterozyklischen, gesättigten oder ungesättigten Ring mit 5 bis 7 Gliedern, und welcher an den verbleibenden Positionen mit einer C1- bis C4-Alkylgruppe, OR14, NHCOR14, COR14, COOR14, worin R14 eine C1- bis C8-Alkylgruppe, ein Halogenatom, CF3 oder OCF3 ist, substituiert sein kann;
oder ein pharmazeutisch akzeptable(s/r) Salz, Ester und Amid davon.

10. Pharmazeutische Zusammensetzung, die irgendeine der folgenden Verbindungen umfasst: 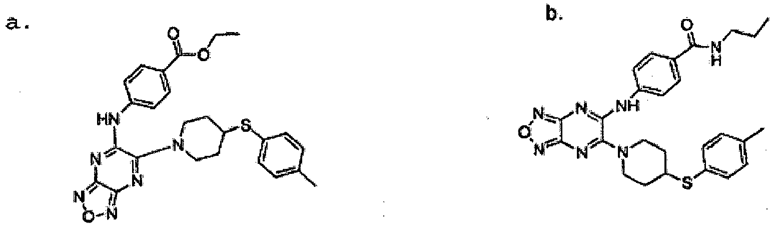 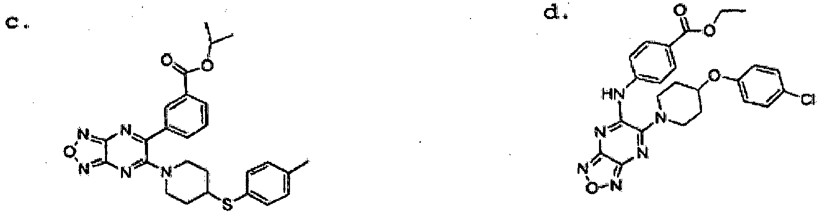 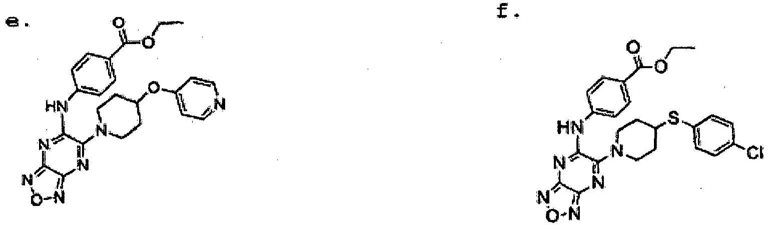 oder ein(en) pharmazeutisch akzeptable(s/n) Salz, Ester und Amid davon; und
einen pharmazeutisch akzeptablen Hilfsstoff.

11. Verfahren zum Screenen einer Verbindung, welche an die Region bindet, die aus Aminosäuren an Positionen 170-199 von SEQ ID NO: 1 und/oder SEQ ID NO: 2 zusammengesetzt ist, wobei eine Verbindungsbibliothek einem ersten Bindungsassay mit einem Protein von SEQ ID NO: 1 und/oder SEQ ID NO: 2 und einem zweiten Bindungsassay mit der Mutante R186A oder R189A des Proteins von SEQ ID NO: 1 und/oder SEQ ID NO: 2 unterworfen wird, und Auswählen der Verbindung(en), deren inhibitorische Wirkung im ersten Bindungsassay größer ist als die im zweiten Bindungsassay.

12. Verfahren zum Bereitstellen einer Verbindung, welche an die Region bindet, die aus Aminosäuren an Positionen 170-199 von SEQ ID NO: 1 und/oder SEQ ID NO: 2 zusammengesetzt ist, wobei eine Verbindungsbibliothek einem ersten Bindungsassay mit einem Protein von SEQ ID NO: 1 und/oder SEQ ID NO: 2 und einem zweiten Bindungsassay mit der Mutante R186A oder R189A des Proteins von SEQ ID NO: 1 und/oder SEQ ID NO: 2 unterworfen wird, und Auswählen der Verbindung(en), deren inhibitorische Wirkung im ersten Bindungsassay größer ist als die im zweiten Bindungsassay, und Synthetisieren der Verbindung oder Bestellen der Verbindung von einem Anbieter.

## Revendications

1. Composé pour une utilisation dans un procédé de traitement de maladies ou de troubles médiés par p38, dans lequel :
le composé se lie à la région composée d'acides aminés au niveau des positions 170 à 199 de SEQ ID NO : 1 de MAPK14 (p38alpha) et/ou SEQ ID NO : 2 de MAPK11 (p38bêta) ; et
dans lequel le composé :
est un peptide de jusqu'à 20 acides aminés de longueur et qui comprend un hexa- ou un heptapeptide ayant l'une quelconque de SEQ ID NO : 3 ou SEQ ID NO : 6 ; ou
est un composé hétérocyclique de la formule générale suivante : 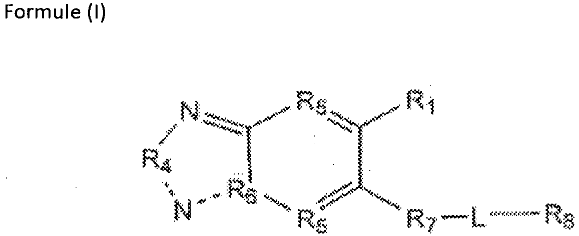 dans lequel
R1 estNR2R3, ou OR2 ;
R2 est un groupe aryle en C5 à C7 ou hétéroaryle en C5 à C7 ;
dans lequel le groupe aryle en C5 à C7 ou hétéroaryle en C5 à C7 est substitué par un ou plusieurs parmi -OR10, -SR10, -NHCOR10, -CONHR10, -COR10, -COOR10, - OCOR10, -NR10R11, -SO2R10, -SO2NR10R11, -CF3, -OCF3 ou -CN, dans lequel R10 et R11 sont indépendamment l'un de l'autre un atome d'hydrogène, un groupe alkyle en C1 à C4, alcényle en C1 à C4 ou alcynyle en C1 à C4; et
dans lequel si R1 est NR2R3, le groupe aryle en C5 à C7 ou hétéroaryle en C5 à C7 peut être lié au N par l'intermédiaire d'une liaison simple ou de NH ;
R3 est un atome d'hydrogène ou un groupe alkyle en C1 à C4;
R4---N---R6 est N=N-N ou O-N=C;
R5 est indépendamment de chaque autre N ou CR11;
dans lequel R11 représente un atome d'hydrogène, un groupe OH, ou SH ;
R7 est un groupe hétérocycloalkyle en C3 à C7 contenant N saturé ou insaturé ;
L est un lieur L, de préférence une liaison covalente simple (de telle façon que R7 est lié directement à R8) ou un groupe alkylène en C1 à C3, dans lequel l'un des groupes CH2 peut être remplacé par O, NH, ou S ;
R8 est un groupe aryle en C5 à C10 ou hétéroaryle en C5 à C10, qui peut être substitué au niveau de la position 4 par un atome d'halogène, un groupe alkyle en C1 à C4, -OR12 ou -SR12 ;
dans lequel R12 est un groupe alkyle en C1 à C4;
ou un sel pharmaceutiquement acceptable, un ester, et un amide de celui-ci ; et les maladies ou les troubles médiés par p38 étant choisis parmi des maladies inflammatoires aiguës et/ou chroniques, la dégénérescence maculaire, une maladie inflammatoire pulmonaire chronique et la maladie d'Alzheimer.

2. Composé pour une utilisation dans un procédé de traitement selon la revendication 1, dans lequel le composé
a) satisfait une structure de pharmacophore dans laquelle cinq points sont définis comme suit :
point A : un fragment hydrophobe/aromatique ;
point B : un fragment hydrophobe/aromatique ;
point C : un point accepteur d'hydrogène ;
point D : un point accepteur d'hydrogène ; et
facultatif : point E : un donneur d'hydrogène,
et dans lequel la relation spatiale entre les points de pharmacophore est spécifiée par des sphères de différents rayons (r) centrées au niveau de leurs coordonnées cartésiennes :
point A (7,99, 34,86, 40,16), r = 1,5 ;
point B (12,16 ; 36,69 ; 37,15), r = 1,6 ;
point C (7,03, 33,05, 40,27), r = 1,2;
point D (9,30, 37,74, 46,62), r = 1,5 ;
point E (12,72, 36,93, 35,072), r = 1,6 ; et
b) se lie à la région composée d'acides aminés au niveau des positions 170 à 199 de SEQ ID NO : 1 de MAPK14 (p38alpha) et/ou SEQ ID NO : 2 de MAPK11 (p38bêta).

3. Composé pour une utilisation dans un procédé de traitement selon la revendication 1, dans lequel le composé a un effet inhibiteur sur la protéine de SEQ ID NO : 1 et/ou de SEQ ID NO : 2, mais dans une moindre mesure sur le mutant R186A ou R189A de la protéine de SEQ ID NO : 1 et/ou SEQ ID NO : 2.

4. Composé pour une utilisation dans un procédé de traitement selon l'une quelconque des revendications 1 à 3, pour une utilisation dans un procédé de traitement ou de prévention de maladies ou de troubles inflammatoires.

5. Composé pour une utilisation dans un procédé de traitement selon l'une quelconque des revendications 1 à 3, pour une utilisation dans un procédé de traitement ou de prévention de la polyarthrite rhumatoïde, de l'ostéoarthrose, de l'arthrite psoriasique, de la douleur inflammatoire et de l'inflammation du système musculosquelettique.

6. Composé pour une utilisation dans un procédé de traitement selon une ou plusieurs des revendications précédentes, le composé ayant une Cl50 de 0,01 nM à 100 µM pour l'inhibition de l'activité de la protéine MAPK p38.

7. Composé pour une utilisation dans un procédé de traitement selon la revendication 6, le composé présentant une Cl50 de 0,1 nM à 10 µM pour l'inhibition de l'activité de la protéine MAPK p38.

8. Composé pour une utilisation dans un procédé de traitement selon la revendication 1, dans lequel
R1 est NR2R3 ;
R2 est un groupe aryle en C6 ou hétéroaryle en C5 à C6, qui est substitué par l'un parmi -OR10, -SR10, -NHCOR10, -CONHR10, -COR10, -COOR10, -OCOR10, - NR10R11, -SO2R10, -SO2NR10R11, -CF3, -OCF3 ou -CN, dans lequel R10 et R11 sont indépendamment l'un de l'autre un atome d'hydrogène, un groupe alkyle en C1 à C4, alcényle en C1 à C4 ou alcynyle en C1 à C4 ;
dans lequel le groupe aryle en C6 ou hétéroaryle en C5 à C6 peut être lié au N par l'intermédiaire d'une liaison simple ou de NH;
R3 est H ;
R4---N---R6 est N=N-N ou O-N=C;
R5 est indépendamment de chaque autre N ou CR11, dans lequel R11 est un atome d'hydrogène ;
R7 est un groupe hétérocycloalkyle en C₆ contenant N saturé ou insaturé ;
L est une liaison covalente simple (de telle façon que R7 est lié directement à R8) ou un groupe alkylène en C1 à C3, dans lequel l'un des groupes CH2 peut être remplacé par O, NH, S ou CO ;
R8 est un groupe aryle en C6 à C10 ou hétéroaryle en C6 à C10.

9. Composé pour une utilisation dans un procédé de traitement selon la revendication 1 ou 8, dans lequel le composé a la formule suivante : 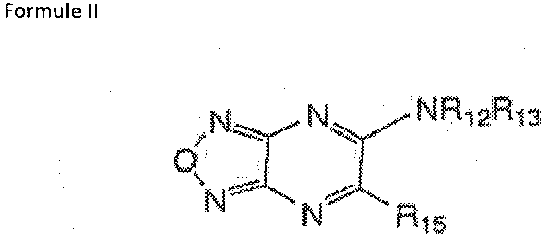 et dans lequel
R12 est un atome d'hydrogène, ou un groupe alkyle en C1 en C4;
R13 est une structure de cycle,
la structure de cycle étant un cycle aryle qui est substitué au niveau des positions 3 ou 4 par CF3, OCF3, OR14, NHCOR14, COR14, CONHR14 ou COOR14, dans lequel R14 est un groupe alkyle en C1 à C8;
ou un cycle hétérocyclique contenant O, N et/ou S, saturé ou non saturé, avec 5 à 7 chaînons de cycle qui est substitué au niveau de la position 3 ou 4 par un atome d'halogène, CF3, ou OCF3, OR14, NHCOR14, COR14, ou COOR14, dans lesquels R14 est un groupe alkyle en C1 à C8, et qui peut être substitué par un atome d'halogène, un groupe CF3, OCF3, alkyle en C1 à C4, OR14, NHCOR14, COR14, ou COOR14, dans lequel R14 est H ou un groupe alkyle en C1 à C8 au niveau des positions restantes ;
dans lequel la structure de cycle ou le cycle hétérocyclique contenant O, N et/ou S peut être lié au N par l'intermédiaire d'une liaison simple ou de NH ;
R15 est un hétérocycle contenant N, avec le N se liant à la structure de cycle oxazolidinone, qui est liée, par l'intermédiaire d'un lieur, à :
un aryle en C6 à C10 ou un cycle phényle qui pourra être substitué au niveau de la position 4 par OR4, NHCOR14, COR14, COOR14, dans lequel R14 est un groupe alkyle en C1 à C8, un atome d'halogène, CF3, ou OCF3, et qui peut être substitué par un groupe alkyle en C1 à C4, un atome d'halogène, OR14, NHCOR14, COR14, COOR14, dans lequel R14 représente un groupe alkyle en C1 à C8, un atome d'halogène, CF3, ou OCF3 au niveau des positions restantes;
ou un cycle hétérocyclique contenant O, N et/ou S, saturé ou non saturé, avec 5 à 7 chaînons de cycle, et qui peut être substitué par un groupe alkyle en C1 à C4, OR14, NHCOR14, COR14, COOR14, dans lequel R14 est un groupe alkyle en C1 à C8, un atome d'halogène, CF3, ou OCF3 au niveau des positions restantes;
ou un sel pharmaceutiquement acceptable, un ester, et un amide de celui-ci.

10. Composition pharmaceutique comprenant l'un quelconque des composés suivants : 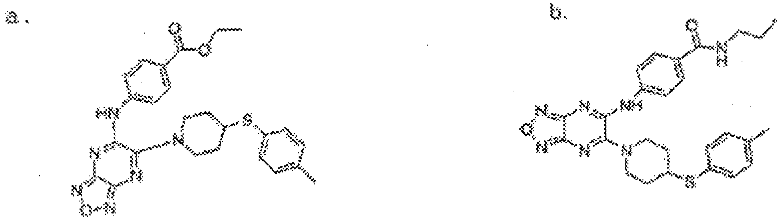 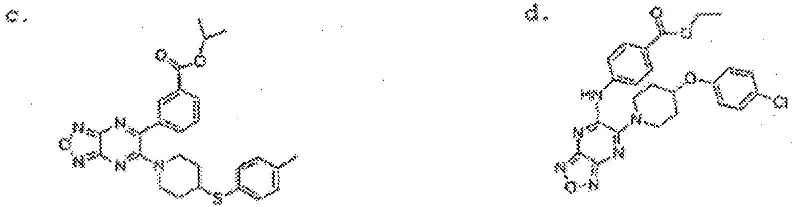 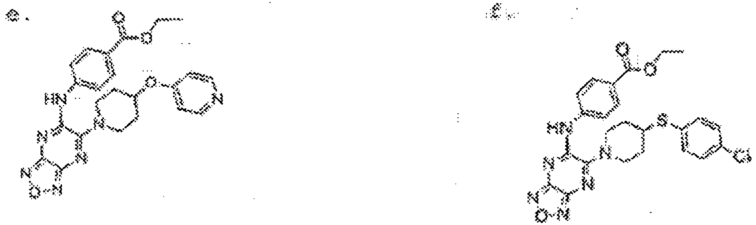 ou un sel pharmaceutiquement acceptable, un ester, et un amide de celui-ci ; et un excipient pharmaceutiquement acceptable.

11. Procédé pour cribler un composé qui se lie à la région composée d'acides aminés au niveau des positions 170 à 199 de SEQ ID NO : 1 et/ou SEQ ID NO : 2, dans lequel une banque de composés est soumise à un premier test de liaison avec une protéine de SEQ ID NO : 1 et/ou SEQ ID NO : 2, et à un second test de liaison avec le mutant R186A ou R189A de la protéine de SEQ ID NO : 1 et/ou SEQ ID NO : 2, et pour sélectionner le ou les composés dont l'action inhibitrice dans le premier test de liaison est supérieure à celle dans le second test de liaison.

12. Procédé pour fournir un composé qui se lie à la région composée d'acides aminés au niveau des positions 170 à 199 de SEQ ID NO : 1 et/ou SEQ ID NO : 2, dans lequel une banque de composés est soumise à un premier test de liaison avec une protéine de SEQ ID NO : 1 et/ou SEQ ID NO : 2, et à un second test de liaison avec le mutant R186A ou R189A de la protéine de SEQ ID NO : 1 et/ou SEQ ID NO : 2, et pour sélectionner le ou les composés dont l'action inhibitrice dans le premier test de liaison est supérieure à celle dans le second test de liaison, et synthétiser du composé ou commander le composé auprès d'un fournisseur.


### SEQUENCE LISTING

<110> Allinky
<120> p38 MAPK inhibitors for the treatment of inflammatory diseases
<130> 161118
<160> 10
<170> PatentIn version 3.4
<210> 1
   <211> 360
   <212> PRT
   <213> Homo sapiens
<220>
   <221> PEPTIDE
   <222> (1)..(360)
   <223> Amino acid Sequence of MAPK 14 (p38alpha)
<400> 1
   Met Ser Gln Glu Arg Pro Thr Phe Tyr Arg Gln Glu Leu Asn Lys Thr
   1               5                   10                  15
   Ile Trp Glu Val Pro Glu Arg Tyr Gln Asn Leu Ser Pro Val Gly Ser
               20                  25                  30
   Gly Ala Tyr Gly Ser Val Cys Ala Ala Phe Asp Thr Lys Thr Gly Leu
           35                  40                  45
   Arg Val Ala Val Lys Lys Leu Ser Arg Pro Phe Gln Ser Ile Ile His
       50                  55                  60
   Ala Lys Arg Thr Tyr Arg Glu Leu Arg Leu Leu Lys His Met Lys His
   65                  70                  75                  80
   Glu Asn Val Ile Gly Leu Leu Asp Val Phe Thr Pro Ala Arg Ser Leu
                   85                  90                  95
   Glu Glu Phe Asn Asp Val Tyr Leu Val Thr His Leu Met Gly Ala Asp
               100                 105                 110
   Leu Asn Asn Ile Val Lys Cys Gln Lys Leu Thr Asp Asp His Val Gln
           115                 120                 125
   Phe Leu Ile Tyr Gln Ile Leu Arg Gly Leu Lys Tyr Ile His Ser Ala
       130                 135                 140
   Asp Ile Ile His Arg Asp Leu Lys Pro Ser Asn Leu Ala Val Asn Glu
   145                 150                 155                 160
   Asp Cys Glu Leu Lys Ile Leu Asp Phe Gly Leu Ala Arg His Thr Asp
                   165                 170                 175
   Asp Glu Met Thr Gly Tyr Val Ala Thr Arg Trp Tyr Arg Ala Pro Glu
               180                 185                 190
   Ile Met Leu Asn Trp Met His Tyr Asn Gln Thr Val Asp Ile Trp Ser
           195                 200                 205
   Val Gly Cys Ile Met Ala Glu Leu Leu Thr Gly Arg Thr Leu Phe Pro
       210                 215                 220
   Gly Thr Asp His Ile Asp Gln Leu Lys Leu Ile Leu Arg Leu Val Gly
   225                 230                 235                 240
   Thr Pro Gly Ala Glu Leu Leu Lys Lys Ile Ser Ser Glu Ser Ala Arg
                   245                 250                 255
   Asn Tyr Ile Gln Ser Leu Thr Gln Met Pro Lys Met Asn Phe Ala Asn
               260                 265                 270
   Val Phe Ile Gly Ala Asn Pro Leu Ala Val Asp Leu Leu Glu Lys Met
           275                 280                 285
   Leu Val Leu Asp Ser Asp Lys Arg Ile Thr Ala Ala Gln Ala Leu Ala
       290                 295                 300
   His Ala Tyr Phe Ala Gln Tyr His Asp Pro Asp Asp Glu Pro Val Ala
   305                 310                 315                 320
   Asp Pro Tyr Asp Gln Ser Phe Glu Ser Arg Asp Leu Leu Ile Asp Glu
                   325                 330                 335
   Trp Lys Ser Leu Thr Tyr Asp Glu Val Ile Ser Phe Val Pro Pro Pro
               340                 345                 350
   Leu Asp Gln Glu Glu Met Glu Ser
           355                 360
<210> 2
   <211> 364
   <212> PRT
   <213> Homo sapiens
<220>
   <221> PEPTIDE
   <222> (1)..(364)
   <223> Peptide Sequence of MAPK 11 (p38beta)
<400> 2
   Met Ser Gly Pro Arg Ala Gly Phe Tyr Arg Gln Glu Leu Asn Lys Thr
   1               5                   10                  15
   Val Trp Glu Val Pro Gln Arg Leu Gln Gly Leu Arg Pro Val Gly Ser
               20                  25                  30
   Gly Ala Tyr Gly Ser Val Cys Ser Ala Tyr Asp Ala Arg Leu Arg Gln
           35                  40                  45
   Lys Val Ala Val Lys Lys Leu Ser Arg Pro Phe Gln Ser Leu Ile His
       50                  55                  60
   Ala Arg Arg Thr Tyr Arg Glu Leu Arg Leu Leu Lys His Leu Lys His
   65                  70                  75                  80
   Glu Asn Val Ile Gly Leu Leu Asp Val Phe Thr Pro Ala Thr Ser Ile
                   85                  90                  95
   Glu Asp Phe Ser Glu Val Tyr Leu Val Thr Thr Leu Met Gly Ala Asp
               100                 105                 110
   Leu Asn Asn Ile Val Lys Cys Gln Ala Leu Ser Asp Glu His Val Gln
           115                 120                 125
   Phe Leu Val Tyr Gln Leu Leu Arg Gly Leu Lys Tyr Ile His Ser Ala
       130                 135                 140
   Gly Ile Ile His Arg Asp Leu Lys Pro Ser Asn Val Ala Val Asn Glu
   145                 150                 155                 160
   Asp Cys Glu Leu Arg Ile Leu Asp Phe Gly Leu Ala Arg Gln Ala Asp
                   165                 170                 175
   Glu Glu Met Thr Gly Tyr Val Ala Thr Arg Trp Tyr Arg Ala Pro Glu
               180                 185                 190
   Ile Met Leu Asn Trp Met His Tyr Asn Gln Thr Val Asp Ile Trp Ser
           195                 200                 205
   Val Gly Cys Ile Met Ala Glu Leu Leu Gln Gly Lys Ala Leu Phe Pro
       210                 215                 220
   Gly Ser Asp Tyr Ile Asp Gln Leu Lys Arg Ile Met Glu Val Val Gly
   225                 230                 235                 240
   Thr Pro Ser Pro Glu Val Leu Ala Lys Ile Ser Ser Glu His Ala Arg
                   245                 250                 255
   Thr Tyr Ile Gln Ser Leu Pro Pro Met Pro Gln Lys Asp Leu Ser Ser
               260                 265                 270
   Ile Phe Arg Gly Ala Asn Pro Leu Ala Ile Asp Leu Leu Gly Arg Met
           275                 280                 285
   Leu Val Leu Asp Ser Asp Gln Arg Val Ser Ala Ala Glu Ala Leu Ala
       290                 295                 300
   His Ala Tyr Phe Ser Gln Tyr His Asp Pro Glu Asp Glu Pro Glu Ala
   305                 310                 315                 320
   Glu Pro Tyr Asp Glu Ser Val Glu Ala Lys Glu Arg Thr Leu Glu Glu
                   325                 330                 335
   Trp Lys Glu Leu Thr Tyr Gln Glu Val Leu Ser Phe Lys Pro Pro Glu
               340                 345                 350
   Pro Pro Lys Pro Pro Gly Ser Leu Glu Ile Glu Gln
           355                 360
<210> 3
   <211> 6
   <212> PRT
   <213> Artificial Sequence
<220>
   <223> Peptide Sequence
<220>
   <221> PEPTIDE
   <222> (1)..(6)
   <223> Peptide Sequence
<400> 3
   Tyr Ser Asn His Gly Leu
   1               5
<210> 4
   <211> 30
   <212> PRT
   <213> Homo sapiens
<220>
   <221> PEPTIDE
   <222> (1)..(30)
   <223> Specific region 170-199 of MAPK14 (p38alpha)
<400> 4
   Gly Leu Ala Arg His Thr Asp Asp Glu Met Thr Gly Tyr Val Ala Thr
   1               5                   10                  15
   Arg Trp Tyr Arg Ala Pro Glu Ile Met Leu Asn Trp Met His
               20                  25                  30
<210> 5
   <211> 30
   <212> PRT
   <213> Homo sapiens
<220>
   <221> PEPTIDE
   <222> (1)..(30)
   <223> Specific region 170-199 of MAPK11 (p38beta)
<400> 5
   Gly Leu Ala Arg Gln Ala Asp Glu Glu Met Thr Gly Tyr Val Ala Thr
   1               5                   10                  15
   Arg Trp Tyr Arg Ala Pro Glu Ile Met Leu Asn Trp Met His
               20                  25                  30
<210> 6
   <211> 7
   <212> PRT
   <213> Artificial Sequence
<220>
   <223> Artificial inhibitory sequence
<220>
   <221> PEPTIDE
   <222> (1)..(7)
   <223> Inhibitory peptide sequence
<400> 6
   Phe Tyr Ser Asn His Gly Leu
   1               5
<210> 7
   <211> 30
   <212> DNA
   <213> Artificial Sequence
<220>
   <223> Foward Primer
<220>
   <221> misc_feature
   <222> (1)..(30)
   <223> Primer
<400> 7
   gctacgtggc cactgcgtgg tacagggctc 30
<210> 8
   <211> 30
   <212> DNA
   <213> Artificial Sequence
<220>
   <223> Reverse Primer sequence
<220>
   <221> misc_feature
   <222> (1)..(30)
   <223> Primer
<400> 8
   agccctgtac cacgcagtgg ccacgtagcc 30
<210> 9
   <211> 32
   <212> DNA
   <213> Artificial Sequence
<220>
   <223> Forward primer
<220>
   <221> misc_feature
   <222> (1)..(32)
   <223> Primer
<400> 9
   cactaggtgg tacgcggctc ctgagatcat gc 32
<210> 10
   <211> 32
   <212> DNA
   <213> Artificial Sequence
<220>
   <223> Reverse primer sequence
<220>
   <221> misc_feature
   <222> (1)..(32)
   <223> Primer
<400> 10
   catgatctca ggagccgcgt accacctagt gg 32